# EUROPÄISCHE PATENTSCHRIFT

(11) **EP 4 129 377 B1**
(45) Veröffentlichungstag und Bekanntmachung des Hinweises auf die Patenterteilung: **28.10.2026**
(21) Anmeldenummer: 22185701.4
(22) Anmeldetag: 19.07.2022
(51) Int. Cl.: A61M 16/00, A61M 16/04, A61M 16/08, A61M 16/20

(54) **BEATMUNGSSYSTEM MIT SPRECHFUNKTION UND VERFAHREN ZUR STEUERUNG EINER SPRACHGASFÖRDERUNG**
VENTILATION SYSTEM WITH SPEAKING FUNCTION AND METHOD FOR CONTROLLING SPEECH GAS DELIVERY
SYSTÈME DE VENTILATION AVEC FONCTION DE PAROLE ET PROCÉDÉ DE COMMANDE D'UNE DISTRIBUTION DE GAZ DE PHONATION

(30) Priorität: 07.08.2021 DE 102021004079
(43) Veröffentlichungstag der Anmeldung: 08.02.2023
(73) Patentinhaber: Löwenstein Medical Technology S.A., 2557 Luxembourg (LU)
(72) Erfinder: Kremeier, Peter, 76149 Karlsruhe (DE); Göbel, Christof, 22457 Hamburg (DE); Schober, Andreas, 60439 Frankfurt am Main (DE)
(74) Vertreter: Löwenstein Medical IP

(56) Entgegenhaltungen:
- EP-A1- 3 495 011
- WO-A2-2008/033732
- WO-A2-2014/091178
- CN-A- 109 172 986
- JP-A- 2009 153 775
- JP-A- 2016 026 542
- JP-U- 3 193 835
- US-A1- 2016 001 110

## Beschreibung

Die vorliegende Offenbarung betrifft ein System zur Beatmung mit einer Sprechfunktion sowie eine Trachealkanüle, welche ein Sprechen während der Beatmung ermöglicht.

Eine häufige Form der Beatmung, insbesondere im klinischen Bereich, ist die Beatmung der Patienten über eine Trachealkanüle. Dazu wird ein Luftröhrenschnitt angewendet, durch welchen die Kanüle in die Luftröhre, in der Regel unterhalb der Glottis, des Patienten eingeführt wird. Um ein Verrutschen und ein Eindringen von Sekret und Pathogene weitestgehend zu verhindern, werden die Kanülen über einen Ballon in der Luftröhre geblockt. Dies verhindert auch ein Strömen von Atemgas außerhalb der Trachealkanüle durch die Glottis, womit ein Sprechen unmöglich wird.

Im Stand der Technik sind Trachealkanülen bekannt, welche nicht geblockt sind und so angesteuert werden, dass der Patient außerhalb der Trachealkanüle ausatmen können. Trachealkanülen ohne Ballon ("Cuff") bergen aber die Gefahr, dass übermäßig viel Sekret und Pathogene in die Lunge des Patienten gelangen können. Für manche geblockten Trachealkanülen ist vorgesehen, dass diese zum Sprechen des Patienten entblockt werden, der Ballon also entleert wird. Diese Methode ist allerdings träge und auch unangenehm für den Patienten.

EP 3 495 011 A1 offenbart ein System, das Patienten, die künstlich beatmet werden, das Sprechen ermöglichen soll, umfassend ein Beatmungsgerät mit einer Druckluftquelle und einer Trachealkanüle sowie einer Atemluftmesseinheit, die den Atemluftstrom und -druck misst und die Daten an eine Steuereinheit überträgt. Die Trachealkanüle umfasst dabei eine zusammenklappbare, luftundurchlässige Innenwand, die einen Teil der Trachealkanüle vom Atemkanal abschneidet, um den Sprechkanal zu bilden. Durch die Steuerung der "Sprechluft"-Zufuhr ist es dem Patienten mit dem System möglich, zu sprechen.

WO 2014/091178 A2 zeigt ein System, das dem Patienten das Sprechen ermöglichen soll mit einem Beatmungsgerät samt Atemgasquelle und einer Trachealkanüle, wobei ein Ventil durch Gasdruck an einem Steuereinlass betätigt wird. Das Beatmungsgas wird einem Patienten durch einen Hauptkanal bereitgestellt und das Sprachgas durch einen Schlauch mit einem kleineren Durchmesser.

CN 109 172 986 A offenbart eine unterstützende Sprachvorrichtung mit einer Belüftungsvorrichtung sowie einer Kanüle mit einem Atemschlauch, der mit einem Beatmungsgerät verbunden ist und einem "Sprechschlauch", der von den Atemwegen zu einem Luftauslass führt.

JP 2009 153775 A offenbart eine Sprachunterstützungsvorrichtung für einen künstlich beatmeten Patienten mit Gasversorgungsabschnitt zum Zuführen von Atemgas zu einem Patienten, einem Endotrachealtubus und einem Beatmungskanal zur Verbindung des Gasversorgungsabschnitts mit dem Endotrachealtubus. Die Sprachunterstützungsvorrichtung weist einen Kanal auf, dessen Ende von der Tracheotomieöffnung in die Luftröhre eingeführt wird, und einen Einführungsschlauch, dessen Ende mit der Versorgungsquelle der Sprachunterstützungsvorrichtung in Verbindung steht.

JP 2016 026542 A offenbart ein System aus einer Tracheostomiekanüle, einem künstlichen Beatmungsgerät und einer Sprachhilfe, wobei die Tracheostomiekanüle eine Röhre für Atemluft und einen Sprachkanal aufweist sowie eine aufblasbare Manschette. Die Röhren sind dabei in einer Doppelrohrstruktur angeordnet, wobei in den Sprachkanal entsprechend der Exspirationsbewegung des Beatmungsgeräts Luft geleitet wird.

JP 3 193835 U offenbart eine Sprachunterstützungsvorrichtung zur Verwendung an einem Gerät zur künstlichen Beatmung mit einem Atemgasschlauch, der mit einer Atemgasquelle verbunden ist, einem Endotrachealtubus und einem Sprachkanal mit einem Ein-Aus-Ventil, das mit der Atmosphäre in Verbindung steht, wenn der Patient ausatmet und von der Atmosphäre getrennt wird, wenn der Patient einatmet.

Aufgabe der beschriebenen Erfindung ist ein System bereitzustellen, welches ein angenehmes und natürlich wirkendes Sprechen des Patienten zu ermöglichen und einen möglichst geringen gerätetechnischen Aufwand mitführt. Zur Lösung der Aufgabe wird ein System nach Anspruch 1 vorgeschlagen.

Die Erfindung betrifft ein System zur Sprachunterstützung eines Patienten, umfassend zumindest ein Beatmungsgerät und ein Patienteninterface wobei das Beatmungsgerät zumindest eine steuerbare Atemgasquelle umfasst und dazu ausgebildet ist die Atemphasen, zumindest aber Inspiration und Exspiration, des Patienten zu erkennen und wobei das Patienteninterface zumindest eine Sprachröhre und eine Atemröhre aufweist und dazu eingerichtet ist über die Sprachröhre Sprachgas zum Patienten zu leiten und über die Atemröhre Atemgas zum und/oder vom Patienten zu leiten, wobei das System dazu eingerichtet ist, dem Patienten zumindest zeitweise in einem Sprach-Modus Sprachgas zur Verfügung zu stellen um das Sprechen zu ermöglichen. Erfindungsgemäß ist das System dadurch gekennzeichnet, dass das System dazu eingerichtet und ausgebildet ist, in einen Standby-Modus zu schalten, während der Sprach-Modus nicht aktiv ist, wobei im Standby-Modus ein Druck und/oder Fluss an Sprachgas in der Sprachröhre anliegt, welcher geringer ist als der Druck und/oder Fluss im Sprach-Modus, wobei das System dazu eingerichtet und ausgebildet ist eine Sprachabsicht des Patienten zu erkennen und nur dann in den Sprach-Modus schaltet, wenn eine Sprachabsicht des Patienten erkannt wird.

In manchen Ausführungsformen ist das System dadurch gekennzeichnet, dass das Patienteninterface eine Trachealkanüle ist.

In manchen Ausführungsformen ist das System dadurch gekennzeichnet, dass das System dazu eingerichtet ist nur während der Exspiration des Patienten in den Sprach-Modus zu schalten.

In manchen Ausführungsformen ist das System dadurch gekennzeichnet, dass das System dazu eingerichtet und ausgebildet ist, während des Standby-Modus einen Druck und/oder Fluss an Sprachgas vorzugeben, bei welchem der Patient die Glottis schließen kann bzw. geschlossen halten kann

In manchen Ausführungsformen ist das System dadurch gekennzeichnet, dass das System dazu eingerichtet ist, dass eine Sprachfunktion aktiviert und deaktiviert werden kann, wobei eine Schaltung in den Sprach-Modus nur bei aktivierter Sprachfunktion ermöglicht wird.

In manchen Ausführungsformen ist das System dadurch gekennzeichnet, dass das System dazu eingerichtet ist, dass bei deaktivierter Sprachfunktion kein Sprachgas zur Sprachröhre gefördert wird.

In manchen Ausführungsformen ist das System dadurch gekennzeichnet, dass das System dazu eingerichtet ist periodisch zwischen dem Standby-Modus und dem Sprach-Modus umzuschalten, wobei das System während der Inspiration des Patienten in den Standby-Modus schaltet und während der Exspiration des Patienten in den Sprach-Modus schaltet.

In manchen Ausführungsformen ist das System dadurch gekennzeichnet, dass das System dazu eingerichtet und ausgebildet ist, die Sprachabsicht des Patienten anhand eines Druckabfalls und/oder Flussanstiegs des Sprachgases in der Sprachröhre zu erkennen.

In manchen Ausführungsformen ist das System dadurch gekennzeichnet, dass das System dazu eingerichtet und ausgebildet ist, die Sprachabsicht des Patienten über zumindest einen Beschleunigungssensor und/oder zumindest ein Mikrophon zu erkennen.

In manchen Ausführungsformen ist das System dadurch gekennzeichnet, dass das Beatmungsgerät dazu eingerichtet und ausgebildet ist Sprachgas und Atemgas zu fördern.

In manchen Ausführungsformen ist das System dadurch gekennzeichnet, dass die Atemgasquelle dazu eingerichtet ist, das Sprachgas und das Atemgas zu fördern.

In manchen Ausführungsformen ist das System dadurch gekennzeichnet, dass das System ein Steuerventil umfasst, welches zumindest mit der Sprachröhre des Patienteninterface gasleitend verbunden ist

In manchen Ausführungsformen ist das System dadurch gekennzeichnet, dass das Steuerventil mit einem Y-Stück verbunden ist, wobei das Y-Stück zumindest mit der Atemröhre des Patienteninterface verbunden ist und durch das Steuerventil sowohl Atemgas als auch Sprachgas geleitet wird.

In manchen Ausführungsformen ist das System dadurch gekennzeichnet, dass das System eine Sprachsteuerung umfasst, wobei die Sprachsteuerung dazu eingerichtet ist das Steuerventil zu steuern

In manchen Ausführungsformen ist das System dadurch gekennzeichnet, dass das System eingerichtet ist, das Ventil so zu schalten, dass zumindest im Sprach-Modus Sprechgas zur Sprachröhre gefördert wird.

In manchen Ausführungsformen ist das System dadurch gekennzeichnet, dass das Ventil so schaltbar ist, dass während des Standby-Modus ein Druck und/oder Fluss an Sprachgas in der Sprachröhre aufrechterhalten wird, wobei der Druck und/oder Fluss geringer ist als der Druck und/oder Fluss des Sprachgases im Sprach-Modus.

In manchen Ausführungsformen ist das System dadurch gekennzeichnet, dass das Ventil und die Sprachsteuerung in das Beatmungsgerät integriert sind.

In manchen Ausführungsformen ist das System dadurch gekennzeichnet, dass das System neben dem Beatmungsgerät zumindest ein Sprachgerät umfasst, wobei das Sprachgerät zumindest eine Gasquelle umfasst, welche dazu eingerichtet ist zumindest zeitweise Sprachgas zu fördern.

In manchen Ausführungsformen ist das System dadurch gekennzeichnet, dass das Sprachgerät eingerichtet zwischen dem Sprach-Modus und dem Standby-Modus umzuschalten.

In manchen Ausführungsformen ist das System dadurch gekennzeichnet, dass das Sprachgerät mit dem Beatmungsgerät verbunden ist.

In manchen Ausführungsformen ist das System dadurch gekennzeichnet, dass das Sprachgerät dazu eingerichtet ist Informationen zu den Atemphasen des Patienten vom Beatmungsgerät zu empfangen und anhand dieser Informationen den Sprachgasstrom zu steuern.

In manchen Ausführungsformen ist das System dadurch gekennzeichnet, dass das Beatmungsgerät dazu eingerichtet ist das Sprachgerät zu steuern.

In manchen Ausführungsformen ist das System dadurch gekennzeichnet, dass das das Sprachgerät an das Beatmungsgerät ankoppelbar ist.

In manchen Ausführungsformen ist das System dadurch gekennzeichnet, dass das Sprachgerät dazu eingerichtet ist die Atemphasen des Patienten, zumindest die Inspiration und Exspiration, zu erkennen und anhand der Atemphasen in den Sprach-Modus oder den Standby-Modus zu schalten.

In manchen Ausführungsformen ist das System dadurch gekennzeichnet, dass das Sprachgerät dazu eingerichtet und ausgebildet ist eine Sprachabsicht des Patienten zu erkennen und bei einer erkannten Sprachabsicht in den Sprach-Modus zu schalten.

In manchen Ausführungsformen ist das System dadurch gekennzeichnet, dass das Sprachgerät als Beatmungsgerät ausgebildet ist.

In manchen Ausführungsformen ist das System dadurch gekennzeichnet, dass das Sprachgerät ein Gebläse umfasst, welches zur Förderung von Sprachgas eingerichtet ist und das Sprachgerät eine Steuereinheit umfasst, welche dazu eingerichtet und ausgebildet ist, dass Gebläse zu steuern.

In manchen Ausführungsformen ist das System dadurch gekennzeichnet, dass das Sprachgerät eine Ventilsteuerung umfasst, welche mit dem Ventil verbunden ist und dazu eingerichtet ist, das Ventil zu schalten.

Die Erfindung betrifft auch ein Verfahren zur Steuerung einer Sprachgasförderung nach Anspruch 20 wobei ein Sprachgasfluss und/oder Sprachgasdruck in einem Sprach-Modus auf einen ersten Wert vorgegeben wird und in einem Standby-Modus auf einen zweiten Wert eingestellt wird, wobei der erste Wert höher ist als der zweite Wert und der erste Wert so eingestellt wird, dass einem Anwender das Sprechen ermöglicht wird und der Sprachgasfluss und/oder Sprachgasdruck in dem Standby-Modus nicht vollständig auf Null abgesenkt wird, wobei die Umschaltung von Standby-Modus in den Sprach-Modus die Erkennung einer Sprachabsicht des Anwenders erfordert.

In manchen Ausführungsformen ist das Verfahren dadurch gekennzeichnet, dass periodisch zwischen dem Sprach-Modus und dem Standby-Modus umgeschaltet wird.

In manchen Ausführungsformen ist das Verfahren dadurch gekennzeichnet, dass ein Umschalten vom Standby-Modus in den Sprach-Modus das Erkennen einer Sprachabsicht voraussetzt, wobei die Sprachabsicht durch einen Abfall des Sprachgasdruckes während des Standby-Modus und/oder einen Anstieg des Sprachgasflusses während des Standby-Modus erkannt wird.

In manchen Ausführungsformen ist das Verfahren dadurch gekennzeichnet, dass die Sprachgasförderung in einem Rechteckprofil und/oder einem abnehmenden Flussprofil erfolgt, wobei die Sprachgasförderung unabhängig von einem Ausatemprofil des Patienten erfolgt.

Die vorliegende Offenbarung betrifft auch ein Patienteninterface zur zumindest teilweisen Einführung in die Luftröhre eines Patienten umfassend zumindest eine Atemröhre und zumindest eine Sprachröhre und zumindest ein Dichtelement wobei durch die Atemröhre und die Sprachröhre unabhängig voneinander ein Gas geleitet werden kann, wobei die Sprachröhre über einen Sprachanschluss mit einer Sprachgasleitung verbunden wird und zumindest zeitweise mit Sprachgas versorgt wird und wobei die Atemröhre über einen Atemanschluss mit einem Y-Stück mit zumindest einer Atemgasleitung verbunden wird und zumindest zeitweise mit Atemgas versorgt wird, wobei die Sprachröhre eine Sprachöffnung aufweist, welche zwischen dem Sprachanschluss und dem Dichtelement angeordnet ist.

In manchen Ausführungsformen ist das Patienteninterface dadurch gekennzeichnet, dass das Patienteninterface eine Trachealkanüle ist.

In manchen Ausführungsformen ist das Patienteninterface dadurch gekennzeichnet, dass die Atemröhre und die Sprachröhre zumindest abschnittsweise parallel zueinander verlaufen.

In manchen Ausführungsformen ist das Patienteninterface dadurch gekennzeichnet, dass die Atemröhre und die Sprachröhre zumindest abschnittsweise parallel in einer gemeinsamen Röhre verlaufen, wobei die Atemröhre und die Sprachröhre durch eine Röhrenwand voneinander getrennt sind.

In manchen Ausführungsformen ist das Patienteninterface dadurch gekennzeichnet, dass die Sprachröhre nach Sprachanschluss und Sprachöffnung durch einen Röhrenverschluss verschlossen ist, sodass durch über den Sprachanschluss in die Sprachröhre eingeleitetes Sprachgas im Wesentlichen nur über die Sprachöffnung entweicht.

In manchen Ausführungsformen ist das Patienteninterface dadurch gekennzeichnet, dass das Dichtelement als Ballon und/oder Cuff ausgebildet ist und eine Ballonleitung in der Röhre neben der Atemröhre und der Sprachöhre geführt wird.

In manchen Ausführungsformen ist das Patienteninterface dadurch gekennzeichnet, dass die Atemröhre und die Sprachröhr über ein Y-Stück in die Röhre geführt werden.

In manchen Ausführungsformen ist das Patienteninterface dadurch gekennzeichnet, dass sowohl der Sprachanschluss als auch der Atemanschluss unmittelbar an und/oder in dem Y-Stück angeordnet sind.

In manchen Ausführungsformen ist das Patienteninterface dadurch gekennzeichnet, dass an der Röhre eine Halterplatte angeordnet ist, welche so ausgebildet und eingerichtet ist, dass das Patienteninterface am Hals des Patienten im Wesentlichen fixierbar ist.

In manchen Ausführungsformen ist das Patienteninterface dadurch gekennzeichnet, dass die Röhre zumindest teilweise in die Luftröhre eines Patienten einführbar ist, wobei über die Atemröhre Atemgas zu und/oder von der Lunge des Patienten geleitet wird, das Dichtelement nach der Glottis des Patienten in Richtung Lunge die Luftröhre im Wesentlichen abdichtet, sodass kein Atemgas in die Luftröhre zwischen das Dichtelement und die Glottis geleitet wird und wobei die Sprachöffnung zwischen Glottis und Dichtelement angeordnet ist, sodass kein Sprachgas an dem Dichtelement vorbei in die Lunge des Patienten gelangt.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Unter einem Beatmungsgerät ist jedwedes Gerät zu verstehen, welches einen Anwender oder Patienten bei der natürlichen Atmung unterstützt, die Beatmung des Anwenders bzw. Lebewesens (z.B. Patient und/oder Neugeborener und/oder Frühgeborene) übernimmt und/oder zur Atemtherapie dient und/oder anderweitig die Atmung des Anwenders bzw. Patienten beeinflusst. Darunter fallen zum Beispiel, aber nicht ausschließend, CPAP- sowie BiLevel-Geräte, Narkose- bzw. Anästhesiegeräte, Atemtherapiegeräte, (klinische, außerklinische oder Notfall-) Beatmungsgeräte, Highflow-Therapiegeräte und Hustenmaschinen. Beatmungsgeräte können auch als Diagnosegeräte für die Beatmung verstanden werden. Diagnosegeräte können dabei allgemein zur Erfassung von medizinischen und/oder atmungsbezogenen Parametern eines Lebewesens dienen. Darunter fallen auch Geräte, welche medizinische Parameter von Patienten in Kombination mit der Atmung oder ausschließlich die Atmung betreffend, erfassen und optional verarbeiten können.

Als Patienteninterface kann, soweit nicht ausdrücklich anders beschrieben, jegliches Peripheriegerät verstanden werden, welches zur Interaktion, insbesondere zu Therapie- oder Diagnosezwecken, der Messeinrichtung mit einem Lebewesen gedacht ist. Insbesondere kann ein Patienteninterface als Maske eines Beatmungsgerätes bzw. eine mit dem Beatmungsgerät verbundene Maske verstanden werden. Diese Maske kann eine Full-Face Maske, also Nase und Mund umschließende, oder eine Nasenmaske, also eine nur die Nase umschließende Maske, sein. Auch Trachealkanülen und sogenannte Nasenbrillen können als Maske beziehungsweise Patienteninterface eingesetzt werden. Auch eine Highflow-Maske kann als Patienteninterface verstanden werden. In manchen Fällen kann das Patienteninterface auch ein einfaches Mundstück, beispielsweise ein Rohr, sein, durch welches das Lebewesen zumindest ausatmet und/oder einatmet. Im Zuge der hier beschriebenen Erfindung sind als Patienteninterface insbesondere Trachealkanülen und zu verstehen, welche, soweit nicht explizit anders genannt, als Synonyme verwendet werden.

Es ist darauf hinzuweisen, dass in manchen Ausführungsformen der Erfindung das Atemgas und das Sprechgas aus der gleichen Quelle stammen. Eine Unterscheidung in Atemgas und Sprechgas findet dann zum Teil erst statt, wenn das Gas zur Sprachröhre und/oder zur Atemröhre des Patienteninterfaces geleitet wird. In manchen Ausführungsformen kann eine Gasquelle dazu ausgebildet sein, sowohl Atemgas als auch Sprachgas zu fördern.

Beispielsweise können in dem erfindungsgemäßen System zwei Beatmungsgeräte angeordnet sein, welche beide dazu geeignet sind, Atemgas zu fördern. Das Gas, welches durch das Beatmungsgerät gefördert wird, welches zur Sprachröhre des Patienteninterface geleitet wird, wird dabei als Sprachgas bezeichnet, kann sich aber auch genauso als Atemgas eignen. Insgesamt wird also primär durch das Ziel des Gases entschieden, ob es als Atemgas oder Sprachgas bezeichnet wird, nicht zwingend anhand der Quelle. Der Ausdruck Gas wird, soweit nicht anders gekennzeichnet, synonym für Atemgas und Sprechgas verwendet.

Als Sprachgas kann jedwedes Gas bzw. Gasmischungen angesehen werden, welche nicht schädlich auf den menschlichen Körper wirken. Insbesondere sind Gase und/oder Gasmischungen als Sprachgas verwendbar, welche allgemein auch als Atemgas nutzbar sind. Insbesondere zählen dazu (künstliche) Luft, beispielsweise Umgebungsluft und/oder Druckluft einer zentralen Druckgasleitung oder einer Druckluftflasche, und Mischungen aus Sauerstoff und Stickstoff und/oder weiteren Gasen. Grundsätzlich ist als Sprachgas auch reiner Stickstoff verwendbar, dieser eignet sich in der Regel nicht als Atemgas.

Das erfindungsgemäße System lässt sich in verschiedenen Ausführungsformen realisieren. In manchen Ausführungsformen stellt das Beatmungsgerät, welches das Atemgas zur Beatmung des Patienten fördert, auch das Gas zur Verfügung, welches durch die Sprachröhre der Trachealkanüle geleitet wird und das Sprechen des Patienten ermöglicht. In anderen Ausführungsformen umfasst das System zumindest ein weiteres Gerät, beispielsweise ein weiteres Beatmungsgerät und/oder ein Gerät über welches ein Gas, welches sich als Sprachgas eignet, zumindest teilweise gesteuert, gefördert werden kann.

In manchen beispielhaften Ausführungsformen umfasst das System ein Beatmungsgerät, welches sowohl die Atemröhre als auch die Sprachröhre der Trachealkanüle mit Atem-/Sprechgas bedient. Das Beatmungsgerät umfasst dazu beispielsweise eine Atemgasquelle. Das Beatmungsgerät ist über eine gasleitendende Verbindung mit einem Y-Stück verbunden, welches wiederum mit der Atemröhre der Trachealkanüle sowie einer Atemabluft des Beatmungsgerätes verbunden ist. Das Beatmungsgerät ist so eingerichtet, dass es die Atemphasen, zumindest Inspiration und Exspiration, erkennt und entsprechend die Gaszufuhr steuert. Während der Inspiration wird so gesteuert, dass Atemgas durch die Verbindung zum Y-Stück und in die Atemröhre der Trachealkanüle gefördert wird. Während der Exspiration wird beispielsweise die Leitung, durch welche das Atemgas zum Patienten gefördert wird, gesperrt, sodass die ausgeatmete Luft des Patienten durch eine gasleitende Verbindung durch das Y-Stück zur Atemabluft geleitet wird. Beispielsweise ist im System, optional im und/oder am Beatmungsgerät, ein zusätzliches Schaltventil angeordnet. Alternativ dazu kann das Schaltventil auch in der gasleitenden Verbindung zwischen Beatmungsgerät und Y-Stück angeordnet werden, durch welche das Atemgas während der Inspiration zum Patienten gefördert wird. Dieses Schaltventil ist so mit der Atemgasquelle verbunden, dass eine Förderung von Gas zur Atemröhre und/oder Sprachröhre der Trachealkanüle geschaltet werden kann. Während der Inspiration des Patienten wird das Schaltventil beispielsweise so geschaltet, dass Atemgas zum Y-Stück und der Atemröhre gefördert wird. Zur Exspiration wird das Schaltventil dann beispielsweise so geschaltet, dass Atemgas bzw. Sprechgas stattdessen zur Sprachröhre der Trachealkanüle gefördert wird. Das Ventil ist beispielsweise als pneumatisches Schaltventil ausgeführt oder als elektrisches Schaltventil.

In manchen Ausführungsformen ist das Flussprofil des Sprachgases, insbesondere während des Sprachmodus, unabhängig vom Exspirationsprofil des Patienten. Die eigentliche Ausatmung des Patienten wird über die Förderung von Sprachgas also nicht simuliert. Stattdessen ist vorgesehen, dass für den Sprachgasfluss zumindest zwischen einem Rechteckprofil und einem abnehmenden Flussprofil wählen kann. Das Rechteckprofil sieht dabei einen im Wesentlichen konstanten Sprachgasfluss vor. Wird dem Patienten beispielsweise nur während der Exspiration das Sprechen durch Leiten von Sprachgas in die Sprachröhre ermöglicht, wird der Sprachgasfluss zu Beginn der Exspiration stark erhöht und bis zum Ende der Exspiration auf einem konstanten Niveau gehalten. Am Ende der Exspiration des Patienten wird der Sprachfluss dann wieder verringert.

Das abnehmende Flussprofil für das Sprachgas sieht vor, dass zu Beginn der Exspiration des Patienten ein Fluss an Sprachgas vorgegeben wird, welcher während der Exspiration konstant abnimmt. Zum bzw. am Ende der Exspiration wird der Sprachgasfluss auch hier wieder auf 0 und/oder ein vorgebbares Basisniveau abgesenkt.

In manchen Ausführungsformen kann vorgesehen sein, dass das Flussprofil der Sprachgasförderung frei konfigurierbar ist. Für manche Ausführungsformen kann auch vorgesehen sein, dass das Sprachgasprofil einem aufgenommenen Exspirationsprofil des Patienten entspricht.

Beispielsweise ist das Ventil auch so schaltbar, dass ein zumindest geringer Druck und/oder Fluss in der Sprachröhre aufrechterhalten werden kann, auch wenn der Patient in der Inspirationsphase ist.

In einer alternativen oder ergänzenden Ausführung des Systems umfasst das System ein Beatmungsgerät sowie ein zweites Gerät, hier Sprachgerät genannt, zur Förderung von Atemgas/Sprechgas. Die beiden Geräte sind so miteinander verbunden, dass das Beatmungsgerät, welches die Beatmung des Patienten steuert und/oder unterstützt, auch das Sprachgerät, welches das Sprechgas fördert, steuert. Das Sprachgerät kann zum Beispiel so ausgeführt sein, dass ein konstanter Fluss und/oder Druck bereitgestellt wird, in diesem Fall wird beispielsweise ein Ein-/Ausschalten des Sprachgerätes bzw. der Gasquelle des Sprachgerätes durch das Beatmungsgerät gesteuert. Das Sprachgerät ist dabei beispielsweise direkt mit dem Sprachanschluss und der Sprachröhre der Trachealkanüle verbunden. Das Beatmungsgerät steuert das Sprachgerät beispielsweise so, dass die Gasquelle des Sprachgerätes während der Inspiration des Patienten kein Gas zur Sprachröhre fördert. Während der Exspiration des Patienten wird dann das Sprachgerät bzw. die Gasquelle des Sprachgerätes eingeschaltet/zugeschaltet, sodass Sprachgas zur Sprachröhre der Trachealkanüle gefördert wird. Es kann auch angedacht sein, dass die Gasquelle des Sprachgases, beispielsweise ein Gebläse, dauerhaft einen geringen Druck (z.B. unter 5 mbar) und/oder Fluss in der Sprachröhre aufrechterhält und den Druck und/oder Fluss zum Ermöglichen des Sprechens erhöht. In einem solchen System ist die Verwendung eines extra Ventils zur Steuerung des Gasflusses zur Sprachröhre nicht nötig.

In manchen Ausführungsformen wird durch das Sprachgerät ein geringer Mindestfluss und/oder Mindestdruck an Sprachgas in der Sprachröhre aufrechterhalten, unabhängig vom Sprechen des Patienten.

In manchen Ausführungsformen ist das Sprachgerät so eingerichtet, dass die Gasquelle so steuerbar ist, dass zumindest zwischen zwei Gasdruck und/oder Gasflussniveaus umgestellt werden kann, beispielsweise vergleichbar mit einem BiLevel-Beatmungsgerät. Beispielsweise wird durch das Beatmungsgerät das Sprachgerät so gesteuert, dass während der Inspiration des Patienten ein geringer Druck/Fluss durch das Sprachgerät zur Verfügung gestellt wird und während der Exspiration des Patienten auf einen höheren Druck/Fluss des Sprachgeräts umgestellt wird, wodurch ein Sprechen des Patienten möglich wird. Der niedrige Druck des Sprachgerätes während der Inspiration des Patienten wird dabei so gewählt, dass der Patient die Glottis leicht und leckagefrei verschließen kann und geschlossen halten kann. Beispielsweise liegt der Druck dazu unter 5 mbar, bevorzugt in einem Bereich von 1 mbar bis 3 mbar. Auch kann das zweite Gerät, also das Sprachgerät, so ausgeführt sein, dass es an das Beatmungsgerät ankoppelbar ist, beispielsweise über eine serielle Schnittstelle.

In manchen Ausführungsformen, insbesondere, wenn das Sprachgerät einen konstanten Fluss/Druck bereitstellt, besteht die Möglichkeit zwischen Sprachgerät und Sprachanschluss der Trachealkanüle ein zusätzliches Ventil anzuordnen. Dieses Ventil ist beispielsweise so eingerichtet, je nach Atemphase des Patienten einen Gasstrom zum Sprachanschluss/zur Sprachröhre der Trachealkanüle zu leiten. Wird kein Sprachgas in der Sprachröhre benötigt, so wird das Ventil so geschaltet, dass der Sprachgasstrom beispielsweise in die Umgebungsluft entweichen kann. Beispielsweise ist dabei an ein pneumatisches Ventil gedacht, welches über ein elektrisches/proportionales Schaltventil geschaltet wird. Die Druckluft zur Steuerung des pneumatischen Ventils wird beispielsweise über die Gasquelle des Sprachgerätes zur Verfügung gestellt.

In manchen Ausführungsformen des Systems ist das Sprachgerät unabhängig von dem Beatmungsgerät. Dabei ist das Sprachgerät so eingerichtet, dass es die Atemphasen des Patienten unabhängig vom Beatmungsgerät erkennen kann und entsprechend Sprachgas zur Sprachröhre der Trachealkanüle fördert. Beispielsweise verfügt das Sprachgerät dazu über einen Druck- und/oder Flusssensor, wobei das Signal bzw. die Messwerte am Y-Stück abgegriffen werden. Auch kann ein Fluss- und/oder Drucksensor am Y-Stück angeordnet sein, dessen Signale zumindest vom Sprachgerät abgenommen werden. Entsprechend der Atemphase steuert das Sprachgerät die Förderung von Sprachgas zur Sprachröhre.

In manchen Ausführungsformen des erfindungsgemäßen Systems ist daran gedacht, dass das Sprachgerät einen Standby-Modus aufweist. In dem Standby-Modus wird durch das Sprachgerät dauerhaft ein geringer Druck und/oder Fluss an Sprachgas aufrechterhalten. Der Druck wird dabei beispielsweise so geregelt, dass der Patient die Glottis leicht geschlossen halten kann, z.B. unter 5 mbar, bevorzugt maximal 3 mbar. Fängt der Patient mit dem Sprechen an bzw. möchte sprechen, so kann das Gerät aus dem Standby-Modus in einen unterstützenden Sprach-Modus wechseln und durch Erhöhen des Druckes/des Flusses das Sprechen des Patienten ermöglichen bzw. unterstützen.

In manchen Ausführungsformen des Systems basiert die Steuerung der Sprachgasförderung, ergänzend oder alternativ zu Erkennung der Atemphasen des Patienten, auf einer Erkennung einer Sprachabsicht und/oder eines Sprechversuches des Patienten. Über das Sprachgerät wird dabei beispielhaft dauerhaft ein geringer Fluss/Druck der Sprachröhre zur Verfügung gestellt, z.B. in einem Bereich von 1 mbar bis 5 mbar, sodass der Patient die Glottis geschlossen halten kann. Dies entspricht beispielsweise einem Standby-Modus bzw. Standby-Zustand. Beispielsweise sind zur Spracherkennung entsprechende Sensoren in der Sprachröhre und/oder am Sprachanschluss angeordnet. Beispielsweise kann über einen Beschleunigungssensor in der Sprachröhre erkannt werden, ob der Patient spricht bzw. sprechen möchte. Alternativ kann hier auch über ein Mikrophon das Sprechen des Patienten erkannt werden. Wird die Sprachabsicht/ein Sprechen erkannt, so wird der Fluss/Druck des Sprachgases entsprechend durch das Sprachgerät erhöht. Auch an eine Spracherkennung über einen Fluss- und/oder Drucksensor, welcher den Druck/Fluss durch die Sprachröhre misst, ist gedacht. Beginnt der Patient mit dem Sprechen, so nimmt der Druck ab (bzw. der Fluss erhöht sich), das Sprachgerät kann also auch entsprechend den nötigen Fluss bereitstellen. Stoppt der Patient das Sprechen, so erhöht sich der Druck bzw. verringert sich der Fluss, entsprechend kann das Sprachgerät den Fluss/Druck wieder auf einen Standby-Wert regeln.

Es ist zudem auch denkbar, dass das Sprachgerät in das Beatmungsgerät integriert ist. Das Beatmungsgerät kann dabei zum Beispiel in ein Beatmungsmodul und ein Sprachmodul unterteilt werden. Das Sprachmodul umfasst beispielsweise zumindest eine Gasquelle, von welcher Sprachgas bei Bedarf zur Sprachröhre der Trachealkanüle gefördert wird. Das Beatmungsmodul ist beispielsweise so eingerichtet, dass es vollständig die Steuerung vom Sprachmodul übernimmt. Alternativ dazu ist das Sprachmodul als eigenständiges Modul eingerichtet, so dass zum Beispiel nur die Energieversorgung und/oder Informationen über die Atemphasen des Patienten vom Beatmungsmodul geliefert werden. In manchen Ausführungsformen lässt sich das Sprachmodul über das Beatmungsmodul zumindest ein- und ausschalten.

Es ist weiter daran gedacht, dass die Höhe des Sprachgasflusses einstellbar ist, beispielsweise um die Lautstärke des Sprechens regulieren zu können. Ein höherer Sprachgasfluss führt beispielsweise zu einer lauteren Stimme während ein geringerer Sprachgasfluss eine leisere Stimme zu Folge hat. Dabei ist zu beachten, dass ein gewisser Schwellwert für den Sprachgasfluss überschritten werden muss, damit eine vernehmbare Stimme/Sprache des Patienten entsteht. Dieser Schwellwert ist insbesondere von der Physiologie des Patienten abhängig.

In manchen Ausführungsformen ist auch daran gedacht, dass das Atemgas und/oder das Sprachgas, optional unabhängig voneinander, angefeuchtet und/oder erwärmt wird. Dazu ist im System zumindest ein Atemgasanfeuchter und/oder Heizung angeordnet. Der Atemgasanfeuchter und/oder die Heizung können in das Beatmungsgerät und/oder das Sprachgerät integriert sein. Auch eine Kopplung mit dem Beatmungsgerät und/oder dem Sprachgerät, beispielsweise über die Atemgasleitung und/oder Sprachgasleitung, ist denkbar. Die Erfindung betrifft auch ein Verfahren zur Steuerung der Förderung von Sprachgas zu einem Anwender/Patient. In einem Verfahrensschritt wird zur Sprachunterstützung bzw. in einem Sprach-Modus der Sprachgasfluss und/oder -druck auf einen ersten Wert geregelt, welcher einem Anwender das Sprechen ermöglicht. In einem weiteren Verfahrensschritt wird der Sprachgasfluss und/oder -druck in einem Standby-Modus auf einen zweiten, niedrigeren Wert geregelt, welcher dem Anwender erlaubt die Glottis zumindest teilweise und/oder größtenteils zu schließen. Erfindungsgemäß erfordert die Umschaltung von Standby-Modus in den Sprach-Modus die Erkennung einer Sprachabsicht des Anwenders. Die Erkennung der Sprachabsicht kann beispielsweise über die Erfassung eines Abfallens des Sprachgasdruckes und/oder eines Anstiegs des Sprachgasflusses während des Standby-Modus erfolgen.

Das Flussprofil der Sprachgasförderung ist beispielsweis unabhängig von dem Flussprofil der Exspiration des Patienten. Zur Sprachgasförderung kann beispielsweise aus verschiedenen, vordefinierten Flussprofilen ausgewählt werden. Unter anderem ist vorgesehen, dass die Sprachgasförderung in einem Rechteckprofil erfolgt. Dabei wird zu Beginn der Exspiration des Patienten der Sprachgasfluss auf ein Flussniveau angehoben, welches während des Sprechens und/oder der Exspiration des Patienten im Wesentlichen konstant bleibt. Zum und/oder am Ende der Exspiration wird der Fluss des Sprachgases dann wieder abgesenkt. Alternativ oder ergänzend kann ein abnehmendes Flussprofil für die Sprachgasförderung vorgesehen sein, bei welchem zu Beginn der Exspiration des Patienten der Sprachgasfluss auf ein Flussniveau angehoben wird und allmählich abnimmt. Zum und/oder am Ende der Exspiration des Patienten wird der Sprachgasfluss dann wieder vollständig abgesenkt. Optional können die Sprachgasflussprofile zumindest teilweise anpassbar sein, beispielsweise in Höhe des Flusses und/oder Stärke der Abnahme. In manchen Ausführungsformen kann zudem vorgesehen sein, dass das Flussprofil der Sprachgasförderung frei konfigurierbar ist.

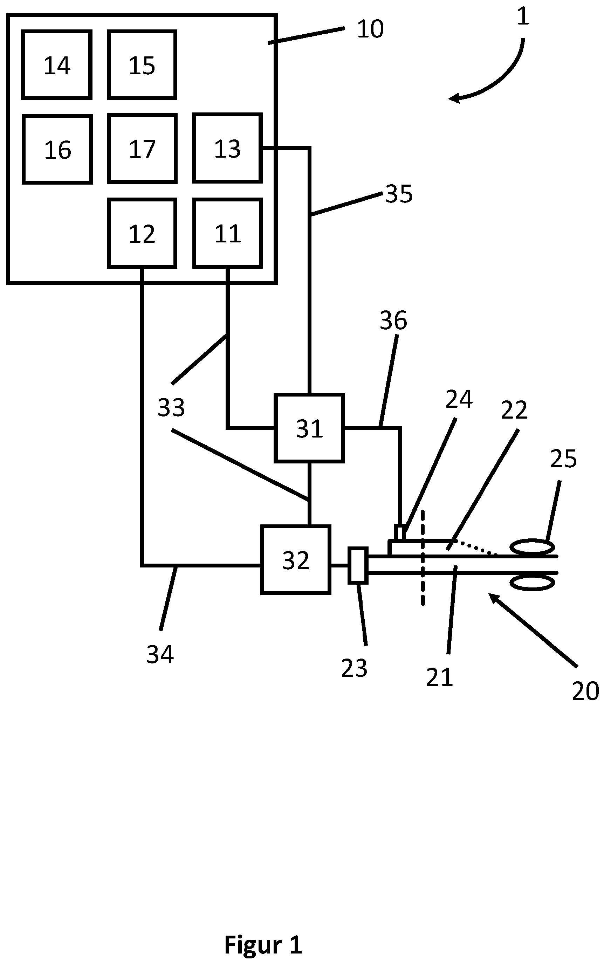

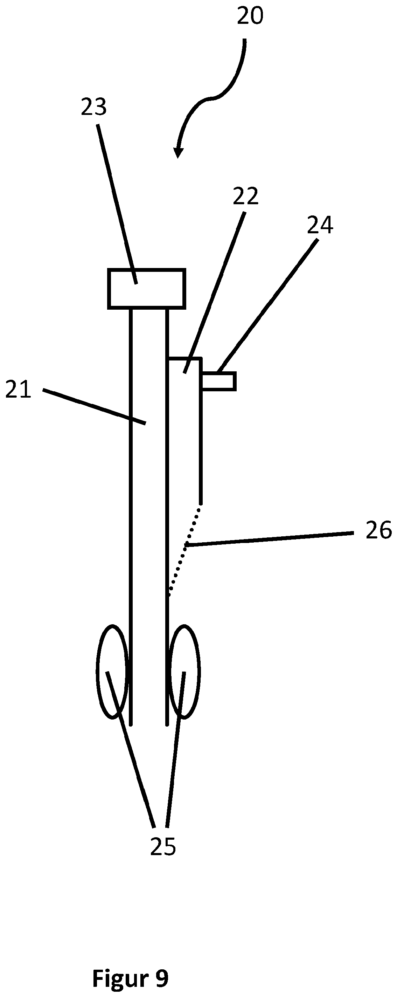

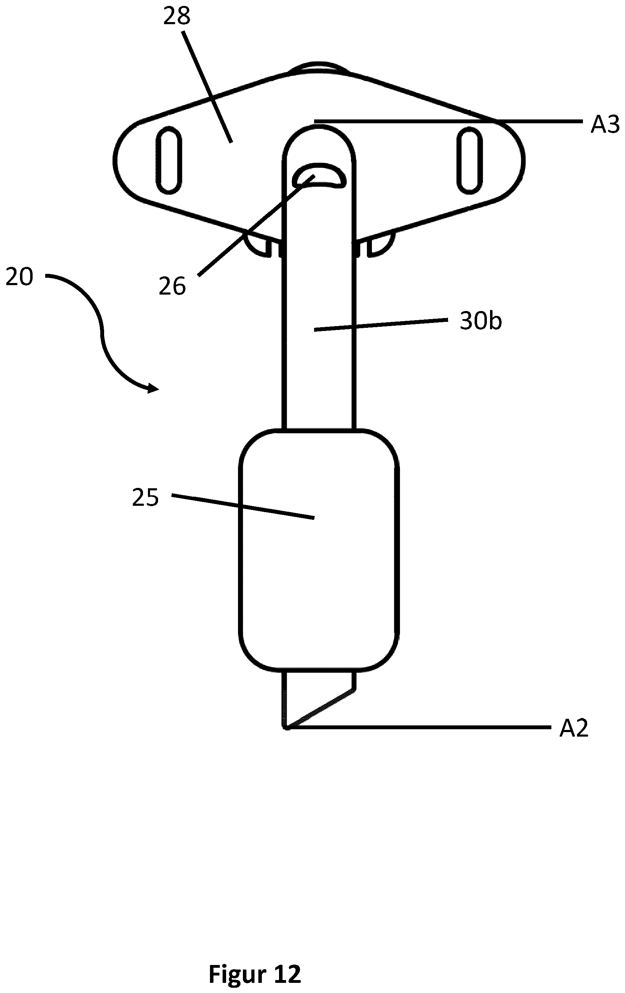

Die Erfindung wird anhand der Figuren 1 bis 13 beispielhaft näher beschrieben. In den Figuren 1 bis 8 werden dabei beispielhafte Ausführungsformen des Systems und deren Funktionsweise näher beschrieben und in den Figuren 9 bis 13 werden beispielhafte Ausführungsformen des Patienteninterface dargestellt.

In Figur 1 ist eine beispielhafte Ausführungsform des Systems 1 zu sehen. Das System 1 umfasst in dieser Ausführungsform ein Beatmungsgerät 10, welches gasleitend mit dem Patienteninterface 20 verbunden ist. Das Patienteninterface 20 ist beispielhaft als Trachealkanüle ausgeführt. Das Patienteninterface 20 umfasst zumindest eine Atemröhre 21, welche zumindest teilweise in die Luftröhre des Patienten eingeführt wird. Die Luftröhre wird um die Atemröhre 21 über zumindest ein Dichtungselement 25 abgedichtet. Durch das Dichtungselement 25 wird sichergestellt, dass Atemgas nur bzw. hauptsächlich durch die Atemröhre 21 zu und von der Lunge des Patienten geleitet wird. Das Dichtungselement 25 ist beispielhaft als Ballon oder Ballonmanschette bzw. als sogenannter Cuff um die Atemröhre 21 ausgebildet. Neben der Atemröhre 21 umfasst das Patienteninterface 20 auch eine Sprachröhre 22. Die Sprachröhre 22 ist beispielhaft in die Trachealkanüle integriert und verläuft zumindest teilweise parallel zur Atemröhre 21. Die Sprachröhre 22 ist beispielsweise so ausgebildet, dass sie eine Öffnung aufweist, welche beispielhaft kurz hinter der Glottis des Patienten aber vor dem Dichtungselement 25 platziert ist. Durch die Sprachröhre 22 wird das Sprachgas geleitet, welches durch die Glottis in den Mundraum entweichen kann, insbesondere dann, wenn der Patient spricht bzw. sprechen möchte. Das Sprachgas simuliert dabei eine Exspiration des Patienten durch die Glottis beim Sprechen. Die Sprachröhre 22 wird über den Sprachanschluss 24 mit einer gasleitenden Verbindung verbunden, in Figur 1 als Sprachgasleitung 36 dargestellt, über welche das Sprachgas zur Sprachröhre 22 geleitet wird. Die Atemröhre 21 ist über einen Atemanschluss 23 gasleitend mit einem Y-Stück 32 verbunden.

Das System 1 ist dabei so eingerichtet und ausgebildet, dass das System 1 zumindest zeitweise in einen Sprach-Modus 63 schalten kann, in welchem dem Patienten über die Sprachröhre 22 Sprachgas zur Verfügung gestellt wird und dem Patienten das Sprechen ermöglicht.

Über das Y-Stück 32 wird während der Inspiration des Patienten Atemgas zur Atemröhre 21 geleitet und während der Exspiration des Patienten von der Atemröhre 21 weggeleitet. Während der Exspiration kann der Patient also durch die Atemröhre 21 und das Y-Stück 32 ausatmen.

Das Beatmungsgerät 10 umfasst zumindest eine Atemgasquelle 11, eine Einrichtung für die Atemabluft 12, eine Sprachsteuerung 13, eine Steuereinheit 14, eine Sensoreinheit 14, eine Auswerteeinheit 16 und eine Benutzerschnittstelle 17. Das Beatmungsgerät 10 ist dazu eingerichtet zumindest teilweise auch die Merkmale eines Sprachgerätes 40 zu umfassen bzw. als ein kombiniertes Beatmungsgerät und Sprachgerät zu fungieren. Das Beatmungsgerät 10 ist beispielhaft dazu eingerichtet sowohl Sprachgas als auch Atemgas zu fördern.

Die steuerbare Atemgasquelle 11 ist dazu ausgebildet, zumindest zeitweise Atemgas zu einem Patienten zu fördern. Die Atemgasquelle 11 kann beispielsweise eine Gebläse- und/oder Ventileinheit sein, welche aus der Umgebungsluft und/oder Gasflaschen das Atemgas generiert. In manchen Ausführungsformen wird das geförderte Atemgas durch zumindest einen Filter geleitet, beispielsweise um zumindest teilweise Staub, Keime, Pathogene, etc. aus dem Atemgas zu filtern. In der in Figur 1 dargestellten Ausführungsform des Systems 1 wird das durch die Atemgasquelle 11 geförderte Gas sowohl als Atemgas als auch als Sprachgas verwendet. In manchen Ausführungsformen umfasst das Beatmungsgerät 10 weiterhin auch Einrichtungen um das Atemgas zu konditionieren, beispielsweise zu befeuchten und/oder zu erwärmen. Dies kann alternativ oder ergänzend auch über ein entsprechendes Modul erreicht werden, welches an das Beatmungsgerät 10 angekoppelt wird oder als zusätzlicher Atemgasanfeuchter in das System 1 integriert wird.

Die Steuereinheit 14 dient beispielsweise der Steuerung des Beatmungsgerätes 10, insbesondere der Atemgasquelle 11 sowie der Ventilsteuerung 13. Unter anderem ist die Steuereinheit 14 dazu ausgebildet, den durch die Atemgasquelle 11 generierten Druck und/oder Fluss zu steuern. Auch kann die Steuereinheit 14 dazu ausgebildet sein, andere Bestandteile/Komponenten des Beatmungsgerätes 10 zu steuern. In manchen Ausführungsformen kann die Steuereinheit 14 auch weiter unterteilt sein und aus mehreren Steuereinheiten bestehen, welche jeweils eine individuelle Einheit und/oder Bestandteil des Beatmungsgerätes 10 steuern. Insbesondere ist die Steuereinheit 14 dazu eingerichtet, das Beatmungsgerät 10 zumindest teilweise automatisch zusteuern. Insbesondere ist die Steuereinheit 14 so programmierbar, dass verschiedene Manöver, während denen zumindest eine Beatmungseinstellung geändert wird, durchzuführen.

Die Sensoreinheit 15 ist dazu eingerichtet, Messwerte, insbesondere Parameter, welche mit einem Atemfluss, einem Atemvolumen, einer Atemfrequenz, einer Inspirations- und/oder Exspirationsdauer, einer Atemkontur, einer Leckage und/oder einem Therapie- bzw. Atemgasdruck in Zusammenhang stehen zu erfassen. Dazu umfasst die Sensoreinheit 15 zumindest einen entsprechend eingerichteten Sensor oder ist mit einem entsprechend eingerichteten Sensor verbunden. Insbesondere ist die Sensoreinheit 15 dazu eingerichtet den Atemgasdruck und/oder den Atemgasfluss zu erfassen. Optional kann die Sensoreinheit 15 auch dazu eingerichtet sein, weitere Parameter/Messwerte wie zum Beispiel Temperatur und/oder Sauerstoffkonzentration und/oder CO2-Konzentrationen des Atemgases und/oder des Blutes zu erfassen. Auch eine Kopplung mit weiteren und/oder externen Sensoren und/oder Sensoreinheiten, wie beispielsweise einem Pulsoxymeter, einer EIT-Einrichtung, etc. ist in manchen Ausführungsformen möglich.

Die von der Sensoreinheit 15 erfassten Parameter und/oder Messwerte werden beispielsweise an die Auswerteeinheit 16 geleitet. Die Auswerteeinheit 16 ist beispielsweise als eine kombinierte Aufbereitungs-, Berechnungs-, Erkennungs- und Auswerteeinheit ausgebildet. Die Auswerteeinheit 16 ist beispielsweise dazu eingerichtet die von beispielsweise der Sensoreinheit 15 erfassten Messwerte und/oder Parameter aufzubereiten und auszuwerten.

Beispielsweise kann die Auswerteeinheit 16 eine Glättung, eine Artefaktbereinigung und/oder ein Downsampling der Messwerte durchführen. Weiter ist die Auswerteeinheit 16 dazu ausgebildet, aus den aufbereiteten Messwerten Signale und/oder Kenngrößen, wie beispielsweise einen Mittelwert, einen Median, ein Perzentil, eine Ableitung, eine Häufigkeitsverteilung, eine Dauer und/oder einen Anteil eines Über- oder Unterschreitens von Schwellenwerten zu berechnen. Die Auswerteeinheit 16 ist weiter dazu eingerichtet, Ereignisse/Zustände wie beispielsweise Alarme, Atemaussetzer, Artefakte, Hustenstöße, Sauerstoff(ent)sättigungen, Asynchronien zwischen Gerät und Anwender und/oder spontane Atemzüge zu erkennen. Insbesondere ist die Auswerteeinheit 16 dazu eingerichtet, die Atemphasen des Patienten zu erkennen, zumindest jedoch die Inspiration und/oder Exspiration. Die Auswerteeinheit 16 ist in manchen Ausführungsformen auch dazu eingerichtet, technische Probleme des Beatmungsgerätes 10 zu erfassen. Technische Probleme können beispielsweise ein niedriger Akkustand, Fehler in der Elektronik, ein defekter Akku, ein defektes Bauteil, ein Stromausfall, ein nicht korrekt funktionierendes Zubehörteil, ein unplausibler Messwert oder ein Verlassen eines erlaubten Temperaturbereichs sein. Die Auswerteeinheit 16 kann bei einem erkannten technischen Problem einen Alarm auf dem Beatmungsgerät 10 und/oder über eine Schnittstelle anzeigen und/oder übermitteln.

In manchen Ausführungsformen umfasst das Beatmungsgerät 10 auch eine, hier nicht dargestellte, Speichereinheit. Dies Speichereinheit ist beispielsweise dazu eingerichtet, Daten und/oder Werte und/oder Informationen zu speichern und/oder zwischen zu speichern. Diese Daten/Werte/Informationen können beispielsweise die durch die Sensoreinheit 15 erfasst und/oder durch die Auswerteeinheit 16 aufbereiteten Werte/Daten/Informationen sein. Auch können in der Speichereinheit beispielsweise Werte/Daten/Informationen hinterlegt werden bzw. sein, beispielsweise durch Eingabe über eine Benutzerschnittstelle 17.

Benutzereingaben, wie zum Beispiel Einstellungen zur Beatmung und/oder Wahl eines Beatmungsprogrammes können beispielsweise über die Benutzerschnittstelle 17 eingegeben werden. Beispielsweise ist die Benutzerschnittstelle 17 als Touchscreen ausgeführt. In manchen Ausführungsformen können alternativ oder ergänzend dazu auch Knöpfe und/oder Schalter und/oder Drehregler, optional zusammen mit einer Anzeige, als Benutzerschnittstelle 17 angeordnet sein.

Die Einrichtung zur Atemabluft 12 ist beispielsweise so eingerichtet, dass hier das ausgeatmete Atemgas des Patienten an die Umgebung abgeführt wird. Dazu ist die Atemabluft 12 beispielsweise über die Atemgasleitung 34 mit dem Y-Stück 32 verbunden. Beispielsweise kann die Atemabluft 12 zumindest teilweise über das Ventil gesteuert werden, welches auch den Druck/Fluss der Atemgasquelle 11 steuert. In manchen Ausführungsformen wird das ausgeatmete Atemgas in der Einrichtung zur Atemabluft 12 vor dem Entweichen in die Umgebungsluft gefiltert und/oder anders gereinigt.

Das Beatmungsgerät 10 ist beispielsweise dazu eingerichtet, je nach Atemphase des Patienten, den Druck und/oder den Fluss des Atemgases auf ein inspiratorisches Niveau anzuheben um die Inspiration des Patienten zu unterstützen und/oder vorzugeben. Um die Exspiration des Patienten zu unterstützen und/oder vorzugeben ist das Beatmungsgerät 10 dazu eingerichtet, den Atemgasfluss und/oder Atemgasdruck auf ein exspiratorisches Niveau abzusenken. In manchen Ausführungsformen wird während der Exspiration die Atemgasleitung 33 so gesperrt, dass im Wesentlichen kein Atemgas vom Y-Stück 32 durch die Atemgasleitung 33 fließt. In manchen Ausführungsformen ist die Pneumatik innerhalb des Beatmungsgerätes 10 so eingerichtet und ausgebildet, dass die Atemgasquelle 11 während der Exspiration einen Atemgasdruck, beispielsweise einen positiven end-exspiratorischen Druck (PEEP) und/oder einen positiven exspiratorischen Atemwegsdruck (EPAP), in der Atemgasleitung 34 aufrecht halten kann.

Während der Exspiration soll zudem dem Patienten das Sprechen ermöglicht werden. Dazu steuert das System 1 so, dass Atemgas bzw. Sprachgas zur Sprachröhre 22 des Patienteninterface 20 gefördert wird. Beispielhaft wird das gleiche Gas, welches als Atemgas durch die Atemgasquelle 11 gefördert wird, als Sprachgas verwendet. Um der Sprachröhre 22 während der Exspiration des Patienten Atem- bzw. Sprachgas zur Verfügung zu stellen, ist in die Atemgasleitung 33 zwischen der Atemgasquelle 11 und dem Y-Stück 32 ein Steuerventil 31 angeordnet. Das Steuerventil 31 ist dazu eingerichtet, den Gasfluss während der Inspiration des Patienten zum Y-Stück 32 und damit zumindest teilweise zur Atemröhre 21 zu leiten. Während der Exspiration des Patienten kann das Steuerventil 31 so geschalten werden, dass ein Gasfluss zur Sprachröhre 22 des Patienteninterface 20 möglich ist. Die Sprachsteuerung 13 ist dabei dazu eingerichtet das Steuerventil 31 zu steuern.

Das Steuerventil 31 kann beispielsweise als pneumatisches und/oder als elektrisches Ventil ausgeführt sein und ist über die Steuerleitung 35 mit der Sprachsteuerung 13 verbunden. Die Sprachsteuerung 13 wird beispielsweise über die Steuereinheit 14 gesteuert. In manchen Ausführungsformen ist die Sprachsteuerung 13 auch dazu eingerichtet, automatisch zu erkennen ob ein Steuerventil 31 mit dem Beatmungsgerät 10 verbunden ist.

Wird eine Exspiration des Patienten erkannt und/oder durch das Beatmungsgerät 10 vorgegeben, so wird durch die Steuereinheit 14 bzw. die Sprachsteuerung 13 das Schaltventil 31 so geschaltet, dass das von der Atemgasquelle 11 geförderte Gas zumindest teilweise zur Sprachröhre 22 geleitet wird und ein Sprechen des Patienten ermöglicht. Wird die Inspiration bzw. eine Atemanstrengung des Patienten erkannt und/oder vorgegeben, so wird das Steuerventil 31 wieder so geschaltet, dass das Atemgas zur Atemröhre 21 geleitet wird.

Das Steuerventil 31 kann beispielsweise auch so angesteuert werden, dass Sprachgas nur dann über die Sprachgasleitung 36 zur Sprachröhre 22 geleitet wird, wenn eine Sprachabsicht des Patienten erkannt wird. Neben verschiedenen sensorische Möglichkeiten, beispielsweise Mikrophone und/oder Beschleunigungssensoren in der Sprachröhre 22, ist dazu auch ein patientennaher Schalter denkbar. Wird ein solcher Schalter, welcher zum Beispiel direkt mit dem Steuerventil 31 oder dem Beatmungsgerät 10 verbunden ist, betätigt, wird das Steuerventil 31 zur Sprachgasleitung 36 geöffnet und Sprachgas wird zur Sprachröhre 22 geleitet, sodass der Patient sprechen kann.

In manchen Ausführungsformen ist das Beatmungsgerät 10 so eingerichtet, dass das Steuerventil 31 so angesteuert wird, dass es einen Gasfluss zur Sprachröhre während der Exspiration des Patienten zulässt, der bereitgestellte Gasfluss und/oder Gasdruck wird aber soweit runtergeregelt, dass der Patient die Glottis geschlossen halten kann, beispielsweise auf einen Druck unter 5 mbar, bevorzugt zwischen 1 mbar und 3 mbar. Beginnt der Patient mit dem Sprechen öffnet sich die Glottis zumindest teilweise woraufhin ein Druckabfall und/oder Flussanstieg in der Sprachröhre detektiert werden kann bzw. wird. Daraufhin kann der bereitgestellte Gasfluss und/oder Gasdruck durch die Sprachröhre soweit hochgeregelt werden, dass das Sprechen des Patienten ermöglicht wird. Beispielsweise wird hier auf einen Gasfluss geregelt, welcher in einem Bereich von 20 l/min bis 60 l/min, bevorzugt in einem Bereich von 30 l/min bis 50 l /min.

In manchen Ausführungsformen ist die Sprachsteuerung 13 als Software- und/oder Firmware-Modul ausgebildet, über welches bestimmte Komponenten des Beatmungsgerätes 10 angesteuert werden können. Beispielsweise kann es möglich sein, dass über die Sprachsteuerung 13 ein elektrischer Signalgeber angesteuert wird, welcher über eine Steuerleitung 35 mit dem Steuerventil 31, beispielsweise als elektrisches Schaltventil ausgebildet, verbunden ist. Alternativ kann auch eine pneumatische Steuereinheit des Beatmungsgerätes 10 angesteuert werden, welches ein als pneumatisches Schaltventil ausgebildetes Steuerventil 31 ansteuert.

In manchen Ausführungsformen kann die Sprachsteuerung 13 auch als an das Beatmungsgerät 10 ankoppelbares Modul ausgebildet sein, welches die Steuersignale zur Steuerung des Steuerventils 31 über das Beatmungsgerät 10, beispielsweise von der Steuereinheit 14, erhält und daraufhin entsprechend das Schaltventil 31 steuert.

In manchen Ausführungsformen weist das Beatmungsgerät 10 eine schaltbare Sprachfunktion auf. Wird die Sprachfunktion eingeschaltet, so wird die Steuerung des Schaltventils 31 über die Sprachsteuerung 13 aktiviert und dem Patienten wird während der Exspiration das Sprechen ermöglicht. Die Sprachfunktion lässt sich beispielsweise über die Benutzerschnittstelle 17 des Beatmungsgerätes 10 aktivieren und deaktivieren. In manchen Ausführungsformen wird die Sprachfunktion automatisch aktiviert, wenn das Beatmungsgerät 10 ein angeschlossenes Steuerventil 31 erkennt. Mit dem Einschalten der Sprachfunktion wird ermöglicht, dass das System in den Sprach-Modus schaltet, in welchem dem Patienten durch Förderung von Sprachgas zur Sprachröhre 22 ein Sprechen ermöglicht wird.

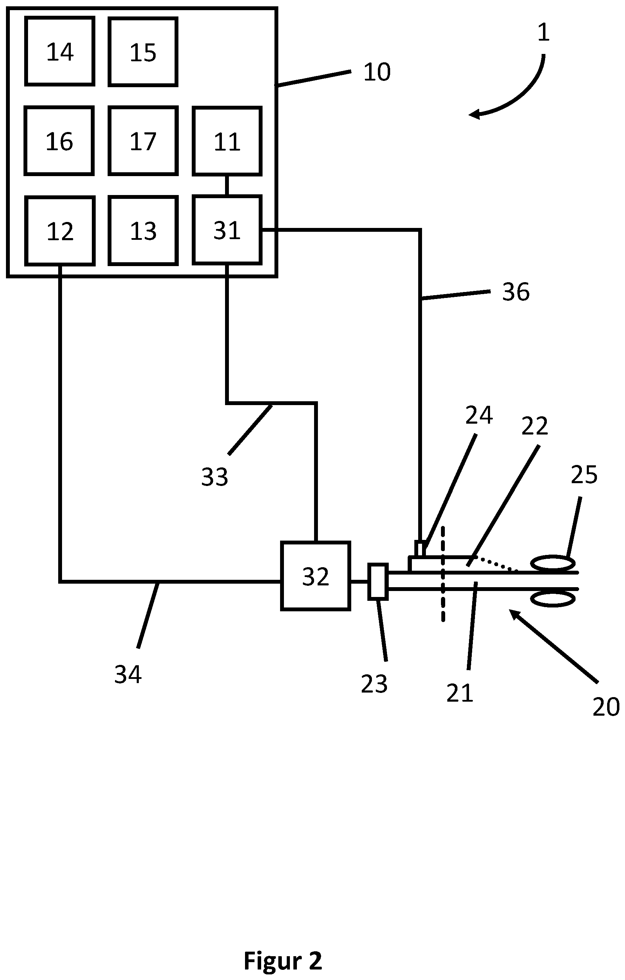

Eine beispielhafte Ausführungsform des Systems 1, bei der das Steuerventil 31 in das Beatmungsgerät 10 integriert ist, ist in Figur 2 schematisch dargestellt. Das Beatmungsgerät 10 ist dabei im Wesentlichen so eingerichtet wie die unter Figur 1 beschriebene Ausführungsform und unterscheidet sich insbesondere darin, dass das Steuerventil 31 innerhalb des Beatmungsgerät 10 angeordnet ist. Die Steuerung des Schaltventils 31 folgt dabei den gleichen Vorgaben wie zu Figur 1 erläutert. Während der Exspiration des Patienten wird das Schaltventil 31, beispielsweise durch die Sprachsteuerung 13, so geschaltet, dass ein Atem- bzw. Sprachgasfluss über die Sprachgasleitung 36 zu Sprachröhre 22 des Patienteninterface 20 geleitet wird. Dadurch wird dem Patienten während der Exspirationsphase das Sprechen ermöglicht. Zur Inspiration wird das Steuerventil 31 hingegen so geschaltet, dass das Atemgas über die Atemgasleitung 33 zum Y-Stück 32 und damit zur Atemröhre 21 des Patienteninterface 20 geleitet wird. In manchen Ausführungsformen ist das Beatmungsgerät 10 so eingerichtet, dass der Gasfluss und/oder Gasdruck für die Inspiration und das Sprechen während der Exspiration jeweils angepasst wird. In manchen Ausführungsformen ist der Atemgasdruck und/oder Atemgasfluss gleich dem Sprachgasdruck und/oder Sprachgasfluss.

Das System 1 ist dabei so eingerichtet und ausgebildet, dass das System 1 zumindest zeitweise in einen Sprach-Modus 63 schalten kann, in welchem dem Patienten über die Sprachröhre 22 Sprachgas zur Verfügung gestellt wird und dem Patienten das Sprechen ermöglicht.

Das Beatmungsgerät 10 übernimmt beispielsweise kombiniert die Funktion eines Beatmungsgerätes und eines Sprachgerätes. Es ist zumindest dazu ausgebildet sowohl Atemgas als auch Sprachgas zu fördern. In manchen Ausführungsformen ist das Beatmungsgerät 10 so ausgebildet, dass gleichzeitig Atemgas und Sprachgas gefördert wird.

In manchen Ausführungsformen (nicht dargestellt) kann das Schaltventil 31 auch in das Y-Stück 32 integriert sein.

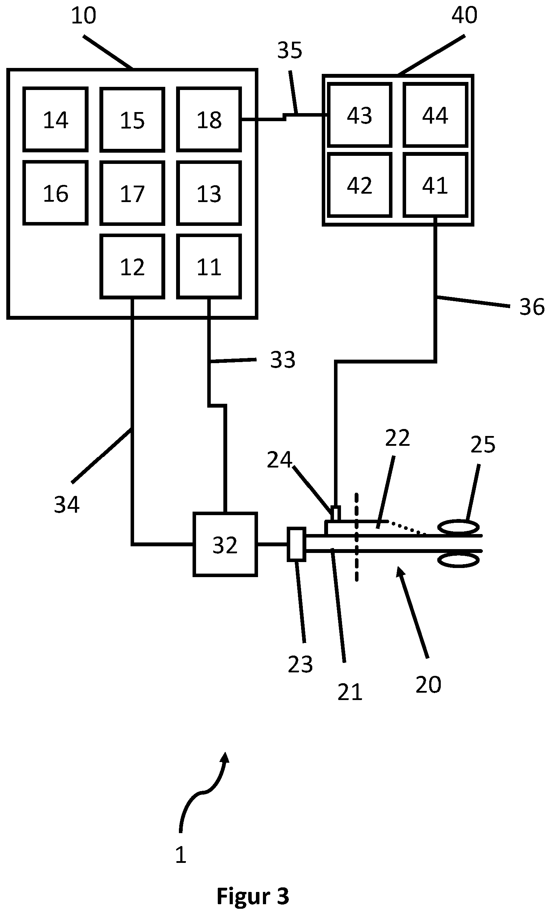

Figur 3 zeigt schematisch eine beispielhafte Ausführungsform des Systems 1, wobei das System 1 neben dem Beatmungsgerät 10 ein Sprachgerät 40 umfasst, welches dazu eingerichtet ist, Sprachgas zur Sprachröhre 22 des Patienteninterface 20 zu fördern. Das Beatmungsgerät 10 entspricht dabei im Wesentlich der in Figur 1 geschilderten Ausführungsform, auf etwaige Unterschiede wird entsprechend eingegangen. Das System 1 ist dabei so eingerichtet und ausgebildet, dass das System 1 zumindest zeitweise in einen Sprach-Modus 63 schalten kann, in welchem dem Patienten über die Sprachröhre 22 Sprachgas zur Verfügung gestellt wird und dem Patienten das Sprechen ermöglicht.

Das Sprachgerät 40 umfasst dabei zumindest eine steuerbare Gasquelle 41, eine Steuereinheit 42 und eine Schnittstelle 43. Die beispielhaft dargestellte Ausführungsform umfasst zudem auch eine Benutzerschnittstelle 44, beispielsweise ausgeführt als Touchscreen und/oder Anzeige mit Bedienelementen. Die Gasquelle 41 ist dazu eingerichtet, ein Sprachgas zu fördern. Beispielsweise kann die Gasquelle 41 eine Gebläse- und/oder Ventileinrichtung sein und Umgebungsluft als Sprachgas ansaugen und/oder ein Gas bzw. ein Gasgemisch aus einer Druckgasquelle (z.B. zentraler Gasanschluss und/oder Gasflaschen) fördern. Die Steuereinheit 42 ist dazu eingerichtet, zumindest die Gasquelle 41 zu steuern. Die Schnittstelle 43 ist dazu eingerichtet eine Verbindung zu dem Beatmungsgerät 10 aufzubauen, wobei die Verbindung beispielsweise eine Kabelverbindung sein kann oder auch eine kabellose Verbindung, beispielsweise eine Funkverbindung. In der in Figur 3 beispielhaft dargestellten Ausführungsform ist die Schnittstelle 43 des Sprachgerätes 40 über eine Steuerleitung 35 mit einer Schnittstelle 18 des Beatmungsgerätes 10 verbunden. Die Benutzerschnittstelle 44 ist dazu eingerichtet, dass Eingaben von einem Benutzer am Sprachgerät 40 eingegeben werden können. Die Benutzerschnittstelle 44 ist beispielsweise dazu eingerichtet, dass zumindest das Sprachgerät 40 über die Benutzerschnittstelle 44 aktiviert/deaktiviert werden kann und/oder ein- und ausgeschaltet werden kann.

Beispielhaft ist die Gasquelle 41 als Gebläse ausgebildet, die Steuerung der Sprachgasförderung kann also ausschließlich durch die Steuerung des Gebläses erreicht werden, ohne das ein Ventil zwischen Gebläse und Sprachröhre 22 nötig ist. Während der Phasen, in denen kein Sprachgas in bzw. zur Sprachröhre 22 geleitet wird, wird das Gebläse deaktiviert. Es kann auch vorgesehen sein, dass in der Sprachröhre 22 ein konstanter Fluss und/oder Druck aufrechterhalten wird, auch wenn sich das System 1 nicht in einem Sprach-Modus 63 befindet. Zwischen Sprach-Modus 63, also dem Zeitraum in dem Sprachgas in die Sprachröhre 22 geleitet wird um dem Patienten das Sprechen zu ermöglichen, und dem Zeitraum außerhalb des Sprach-Modus 63 wird das Gebläse nicht aktiviert bzw. deaktiviert, sondern die Förderleistung wird erhöht bzw. verringert.

Das Sprachgerät 40 ist über die Sprachgasleitung 36 mit der Sprachröhre 22 des Patienteninterface 20 verbunden. Über die Gasquelle 41 wird Sprachgas gefördert und durch die Sprachgasleitung 36 zum Patienteninterface 20 geleitet um dem Patienten zumindest zeitweise das Sprechen zu ermöglichen. Die Förderung von Sprachgas durch das Sprachgerät 40 wird dabei durch das Beatmungsgerät 10 gesteuert bzw. bestimmt. Das heißt, dass das Beatmungsgerät 10 vorgibt, wann und/oder welche Menge an Sprachgas durch das Sprachgerät 40 gefördert und/oder an die Sprachröhre 22 gleitet wird. Dazu ist das Beatmungsgerät 10 über die Steuerleitung 35, welche alternativ oder ergänzend auch eine kabellose Verbindung sein kann, mit dem Sprachgerät 40 verbunden. Die Steuereinheit 42 des Sprachgerätes 40 ist dazu eingerichtet, die Steuersignale des Beatmungsgerätes 10 entsprechend umzusetzen und die Gasquelle 41 entsprechend der Vorgaben des Beatmungsgerätes 10 zu steuern.

Die Vorgaben des Beatmungsgerätes 10 können entsprechend der Ausführung des Sprachgerätes 40 angepasst sein. Im Falle eines Sprachgerätes 40, welches lediglich dazu eingerichtet ist einen konstanten, gegebenenfalls vorgebbaren Gasdruck und/oder Gasfluss bereitzustellen, kann das Beatmungsgerät 10 beispielsweise vorgeben, dass die Gasquelle 41 während der Exspiration des Patienten aktiviert wird und Sprachgas fördert. Ist die Gasquelle 41 beispielsweise als Gebläse ausgebildet, so kann die Sprachgasförderung über eine Ein-/Ausschalten des Gebläses gesteuert werden. Bei einer als Ventileinrichtung ausgeführten Gasquelle 41 ist beispielweise ein Öffnen/Schließen der Ventileinrichtung eine mögliche Steuervariante. Beispielsweise ist das Sprachgerät 40 in manchen Ausführungsformen ein CPAP-Beatmungsgerät.

In manchen Ausführungsformen wird das Sprachgerät 40 nicht direkt durch das Beatmungsgerät 10 gesteuert, sondern es werden Informationen zu den Atemphasen, insbesondere Inspiration und Exspiration, vom Beatmungsgerät 10 an das Sprachgerät 40 weitergegeben, worauf das Sprachgerät 40 selbstständig eine Versorgung mit Sprachgas während der Exspiration des Patienten ansteuert.

In manchen Ausführungsformen ist das Sprachgerät 40 so eingerichtet und ausgebildet, dass ein variabler Sprachgasstrom generiert werden kann. Beispielsweise ist das Sprachgerät 40 dazu ausgebildet im Wesentlichen zwischen zwei Gasdruck- und/oder Gasflussniveaus umzuschalten, ähnlich einem BiLevel-Beatmungsgerät. Beispielsweise ist das Sprachgerät 40 in manchen Ausführungsformen ein BiLevel-Beatmungsgerät. Das Beatmungsgerät 10 übermittelt über die Steuerleitung 35 Informationen zu den Atemphasen des Patienten. Während der Exspiration des Patienten steuert das Sprachgerät 40 die Gasquelle 41 so an, dass ein gewisser Sprachgasfluss durch die Sprachröhre 22 erreicht wird, sodass dem Patienten das Sprechen ermöglicht wird. Zur Inspiration des Patienten regelt das Sprachgerät 40 den Sprachgasfluss und/oder den Sprachgasdruck soweit runter, dass der Patient die Glottis leicht verschließen kann und/oder leicht geschlossen halten kann und/oder die Glottis zumindest teilweise bzw. größtenteils schließen kann. Beispielsweise wird der Sprachgasdruck während der Inspiration des Patienten auf unter 5 mbar geregelt, bevorzugt auf einen Wert zwischen 1 mbar und 3 mbar.

Insbesondere bei einer Ausführung des Sprachgeräts 40 mit einer steuerbaren Gasquelle 41, welche dazu eingerichtet ist zwischen zumindest zwei Druck- und/oder Flussniveaus umzuschalten, ist eine Art Standby-Modus und ein Sprach-Modus möglich. Beispielsweise kann dazu ein Gebläse als Gasquelle 41 eingesetzt werden. Im Standby-Modus wird durch das Sprachgerät 40 ein im Wesentlichen konstanter, niedriger Druck in der Sprachröhre 22 vorgegeben, sodass der Patient die Glottis zumindest teilweise geschlossen halten kann. Das Sprachgerät 40 ist weiter dazu eingerichtet, beispielsweise über entsprechende Sensoreinheiten und Auswerteeinheiten, eine Sprachanstrengung/Sprachabsicht des Patienten festzustellen. Beispielsweise kann eine Sprachanstrengung des Patienten dadurch festgestellt werden, dass dieser zum Sprechen die Glottis öffnet und es dadurch zu einem schlagartigen Druckabfall in der Sprachröhre 22 kommt, welcher durch das Sprachgerät 40 registriert wird. Wird eine Sprachabsicht festgestellt, ist das Sprachgerät 40 dazu eingerichtet vom Standby-Modus in den Sprach-Modus zu wechseln. Für den Sprach-Modus regelt das Sprachgerät 40 den Druck und/oder Fluss des Sprachgases soweit hoch, dass dem Patienten das Sprechen ermöglicht wird. Beispielsweise ist die Umschaltung vom Standby-Modus in den Sprach-Modus auf die Exspiration des Patienten limitiert. Während der Inspiration des Patienten kann das Sprachgerät 40 dann also nicht vom Standby-Modus auf den Sprach-Modus umschalten und einen erhöhten Sprachgasfluss und/oder -druck bereitstellen um das Sprechen des Patienten zu ermöglichen. In Ausführungsformen, in denen das Beatmungsgerät 10 die gesamte Steuerung des Sprachgeräts 40 übernimmt, kann die Sprachabsicht beispielsweise auch durch das Beatmungsgerät 10 ermittelt werden.

Das durch die Gasquelle 41 erzeugte Flussprofil während des Sprach-Modus 63 kann beispielsweise ein Rechteckprofil sein. Um das Sprechen des Patienten zu ermöglichen wird dazu beispielsweise zu Beginn der Exspiration des Patienten der Fluss des Sprachgases angehoben, beispielsweise auf einen zuvor definierten Wert, welcher dann über die Zeitdauer der Exspiration des Patienten im Wesentlichen konstant bleibt. Zum und/oder am Ende der Exspiration des Patienten wird der Flusswert der Sprachgasflusses dann wieder abgesenkt. Neben einem konstanten Sprachgasfluss während der Exspiration des Patienten bzw. der Zeitdauer des Sprach-Modus 63, kann auch ein abnehmender Sprachgasfluss vorgesehen und/oder auswählbar sein. Der Sprachgasfluss ist als unabhängig zum Flussprofil der Exspiration des Patienten zu sehen. Das Flussprofil der Exspiration des Patienten wird durch das Flussprofil der Sprachgasförderung also nicht nachgeahmt bzw. simuliert.

Neben der Übermittlung von Steuersignalen und/oder Informationen zu den Atemphasen des Patienten vom Beatmungsgerät 10 an das Sprachgerät 40 ist auch eine Energieversorgung des Sprachgeräts 40 über die Steuerleitung 35 denkbar. In manchen Ausführungsformen ist das Sprachgerät 40 an das Beatmungsgerät 10 ankoppelbar, beispielsweise kann das Sprachgerät 40 an das Beatmungsgerät 10 angesteckt werden, zum Beispiel als Zusatzmodul.

Das Beatmungsgerät 10 ist beispielsweise so eingerichtet und ausgebildet, dass über die Benutzerschnittstelle 17 die Sprachfunktion aktiviert und deaktiviert werden kann. Bei aktivierte Sprachfunktion wird das Sprachgerät 40 eingeschaltet und/oder das Beatmungsgerät 10 steuert das Sprachgerät 40. In manchen Ausführungsformen ist das Sprachgerät 40 bei Verbindung mit dem Beatmungsgerät 10 immer eingeschaltet, um Sprachgas zu fördern, beispielsweise im Sprach-Modus, muss über das Beatmungsgerät 10 zunächst die Sprachfunktion aktiviert werden. Beispielsweise kann so gewählt werden, ob grundsätzlich eine Sprachunterstützung erwünscht ist oder nicht. In manchen Ausführungsformen wird die Sprachfunktion automatisch aktiviert, sobald das Beatmungsgerät 10 erkennt, dass ein Sprachgerät 40 verbunden wird. Beispielsweise wird eine manuelle Deaktivierung des Sprachgerätes 40 nicht verhindert.

Ferner kann auch vorgesehen sein, dass das Sprachgerät 40 eigenständig den Beginn und das Ende der Exspiration des Patienten ermittelt, beispielsweise über Sensoren und eine im Sprachgerät 40 angeordnete Auswerteeinheit, welche dazu ausgebildet ist die Sensordaten entsprechend auszuwerten.

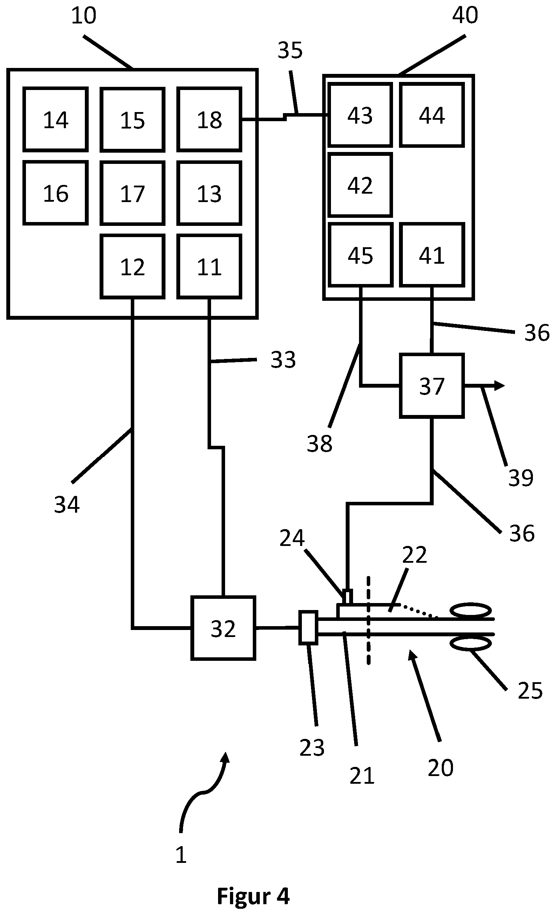

In Figur 4 ist eine weitere beispielhafte Ausführungsform des Systems 1 schematisch dargestellt. Das System 1 ist im Wesentlichen so konfiguriert wie die unter Figur 3 beschriebene Ausführung, wobei in der Sprachgasleitung 36 zwischen Sprachröhre 22 und Sprachgerät 40 ein Steuerventil 37 angeordnet ist. Über dieses Steuerventil 37 lässt sich beispielsweise der Sprachgasstrom, beispielsweise in Form von Druck und/oder Fluss, steuern. Das Steuerventil 37 ist dazu beispielhaft über eine Steuerleitung 38 mit einer Ventilsteuerung 45 im Sprachgerät 40 verbunden. Das Steuerventil 37 kann beispielsweise als pneumatisches Ventil ausgeführt sein, wobei der Steuerdruck, welcher über die Steuerleitung 38 zum Steuerventil 37 geleitet wird, durch die Gasquelle 41 generiert wird und über die Ventilsteuerung 45 eingestellt wird. Die Ventilsteuerung 45 umfasst dabei beispielsweise ein Proportionalventil. Während der Exspiration des Patienten kann das Steuerventil 37, beispielsweise als 3/2-Wege-Ventil ausgeführt, so geschaltet werden, dass Sprachgas zur Sprachröhre 22 des Patienteninterfaces 20 geleitet wird. Während der Inspiration des Patienten wird das Steuerventil 37 dann so geschaltet, dass das Sprachgas zumindest teilweise über die Abluft 39 in die Umgebung entweicht. Das System 1 ist dabei so eingerichtet und ausgebildet, dass das System 1 zumindest zeitweise in einen Sprach-Modus 63 schalten kann, in welchem dem Patienten über die Sprachröhre 22 Sprachgas zur Verfügung gestellt wird und dem Patienten das Sprechen ermöglicht.

Die Ventilsteuerung 45 wird beispielsweise über die Sprachsteuerung 13 des Beatmungsgeräts 10 gesteuert, es ist also eine indirekte Steuerung des Steuerventils 37 über die Sprachsteuerung 13 möglich. Beispielsweise werden die Steuerungsbefehle von der Sprachsteuerung 13 erzeugt/ausgegeben, über die Schnittstelle 18 an das Sprachgerät 40 übertragen und von der Ventilsteuerung 45 umgesetzt.

Die in Figur 4 dargestellte Ausführungsform ist beispielsweise eine alternative oder ergänzende Steuerform in einem System, welches sowohl ein Sprachgerät 40 als auch ein Beatmungsgerät 10 umfasst. Das Sprachgerät 40 ist dabei beispielsweise so eingerichtet und ausgebildet, dass ein im Wesentlichen konstanter Sprachgasstrom generiert wird. Anstatt die Gasquelle 41 während der Inspiration zu deaktivieren und während der Exspiration zu aktiveren ist durch das Steuerventil 37 ein konstanter Betrieb der Gasquelle 41 möglich, wobei über das Steuerventil 37 gesteuert wird, ob Sprachgas zur Sprachröhre geleitet wird.

In manchen Ausführungsformen ist das Steuerventil 37 in das Sprachgerät 40 integriert. In manchen Ausführungsformen wird das Steuerventil 37 auch direkt über das Beatmungsgerät 10 gesteuert, beispielsweise über die Sprachsteuerung 13. Wird das Steuerventil 37 über das Beatmungsgerät 10 bzw. die Sprachsteuerung 13 gesteuert, so ist der Einsatz eines simplen Sprachgerätes 40 möglich, welches einen konstanten Druck/Fluss bereitstellt. Die Zufuhr von Sprachgas an die Sprachröhre 22 wird dann ausschließlich über das Beatmungsgerät 10 und das Steuerventil 37 geregelt.

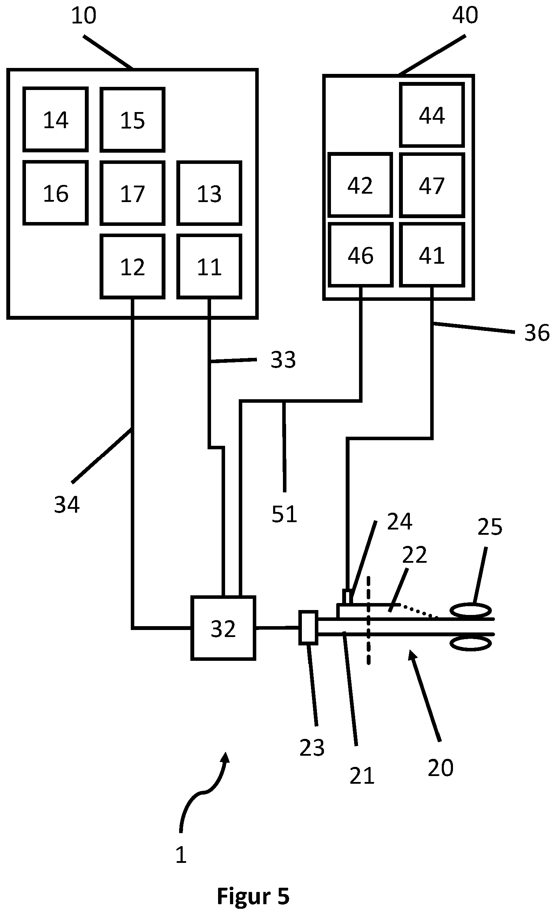

Eine weitere beispielhafte Ausführungsform des Systems 1 ist in Figur 5 dargestellt. Das Beatmungsgerät 10 und das Sprachgerät 40 sind im Wesentlichen so aufgebaut und ausgebildet wie in den vorhergehend beschriebenen Ausführungsformen beschrieben. Im Unterschied zu den vorhergehend beschriebenen Ausführungsformen des Systems 1 ist das Sprachgerät 40 unabhängig von dem Beatmungsgerät 10. Das Beatmungsgerät 10 übermittelt also beispielsweise keine Daten und/oder Informationen zu den Atemphasen oder steuert das Sprachgerät 40 in einer anderen Form. Auch wird von dem Beatmungsgerät 10 in der in Figur 5 dargestellten Ausführungsform die Förderung von Sprachgas zur Sprachröhre 22 des Patienteninterface 20 nicht gesteuert. Das von dem Beatmungsgerät 10 unabhängige Sprachgerät 40 ist beispielsweise so eingerichtet, dass eine Sprachfunktion aktiviert und deaktiviert werden kann. Bei einer aktivierten Sprachfunktion wird dem Sprachgerät 40 grundsätzlich ermöglicht, in den Sprach-Modus zu schalten und ein Sprechen des Patienten zu ermöglichen. In manchen Ausführungsformen wird durch deaktivieren der Sprechfunktion auch der Standby-Modus ausgeschaltet.

Das Sprachgerät 40 ist so eingerichtet und ausgebildet, dass es, beispielhaft über eine Sensoreinheit 46, die Atemphasen des Patienten bestimmt. Dazu greift die Sensoreinheit 46 auf Druck- und/oder Flussdaten des Atemgases über das Y-Stück 32 zu. In manchen Ausführungsformen ist dazu ein Druck- und/oder Flusssensor im Y-Stück 32 angeordnet, welcher die Sensorverbindung 51 mit der Sensoreinheit 46 des Sprachgerätes 40 verbunden ist.

In manchen Ausführungsformen ist der Druck- und/oder Flusssensor im Sprachgerät 40 selbst angeordnet, sodass über die Sensorverbindung 51 eine pneumatische Verbindung zum Y-Stück 32 hergestellt wird.

Das Sprachgerät 40 ermittelt selbstständig, beispielsweise über eine eigene interne Auswerteeinheit 47, welche zumindest teilweise vergleichbar mit der Auswerteeinheit 15 des Beatmungsgerätes 10 ist, die Atemphasen des Patienten - unabhängig davon, ob diese durch das Beatmungsgerät 10 vorgegeben werden oder auf einer Atemanstrengung des Patienten beruhen. Anhand der registrierten Atemphasen steuert das Sprachgerät 40 selbstständig die Förderung von Sprachgas. Beispielsweise wird während der Exspirationsphase ein Sprachgasfluss und/oder -druck bereitgestellt, welcher das Sprechen des Patienten ermöglicht. Während der Inspiration des Patienten hingegen wird beispielsweise der Fluss und/oder Druck vom Sprachgas soweit heruntergeregelt, dass der Patient die Glottis zumindest teilweise bzw. größtenteils verschließen kann bzw. geschlossen halten kann. In manchen Ausführungsformen wird während der Inspiration des Patienten ein so geringer Fluss und/oder Druck an Sprachgas durch das Sprachgerät 40 vorgegeben, dass der Patient die Glottis gegebenenfalls nicht vollständig schließen kann, aber dennoch kein unangenehmes Gefühl entsteht. Beispielsweise wird der Sprachgasdruck während der Inspiration des Patienten auf unter 5 mbar geregelt, bevorzugt unter 3 mbar aber über 1 mbar.

Auch in der in Figur 5 dargestellten Ausführungsform kann ein Standby-Modus vorgesehen sein. Dazu gibt das Sprachgerät 40 während des Standby-Modus einen geringen Druck und/oder Fluss an Sprachgas vor. Öffnet der Patient die Glottis zum Sprechen, so ist das Sprachgerät 40 dazu eingerichtet, den Druckabfall und/oder Flussanstieg zu registrieren und, beispielsweise ab Überschreiten (Flusswert) und/oder Unterschreiten (Druckwert) eines ggf. einstellbaren Schwellwerts, als Sprachabsicht zu erkennen. Wird die Sprachabsicht erkannt, stellt das Sprachgerät 40 vom Standby-Modus in einen Sprach-Modus um und regelt die Förderung von Sprachgas so, dass ein Sprechen des Patienten möglich ist. Beispielsweise regelt das Sprachgerät 40 dazu den Fluss auf einen Wert zwischen 10 l/min und 80 l/min, bevorzugt zwischen 20 l/min und 60 l/min, mehr bevorzugt zwischen 30 l/min und 50 l/min. Stoppt der Patient das Sprechen erhöht sich zumindest der Druck in der Sprachröhre. Das Sprachgerät 40 schaltet daraufhin zurück in den Standby-Modus. Der Fluss des Sprachgases kann in manchen Ausführungsformen auch anhand einer (grob) geregelten Sprachlautstärke vorgegeben werden. Beispielsweise ist eine Einteilung in leise - mittel - laut möglich, wobei mit der Einstellung "leise" ein geringerer Sprachgasfluss zusammenhängt als mit "mittel" und "laut", und die Einstellung "laut" den höchsten Sprachgasfluss bedeutet.

Das System 1 ist dabei so eingerichtet und ausgebildet, dass das System 1 zumindest zeitweise in einen Sprach-Modus 63 schalten kann, in welchem dem Patienten über die Sprachröhre 22 Sprachgas zur Verfügung gestellt wird und dem Patienten das Sprechen ermöglicht.

In manchen Ausführungsformen ist das Sprachgerät 40 ebenfalls als Beatmungsgerät ausgeführt.

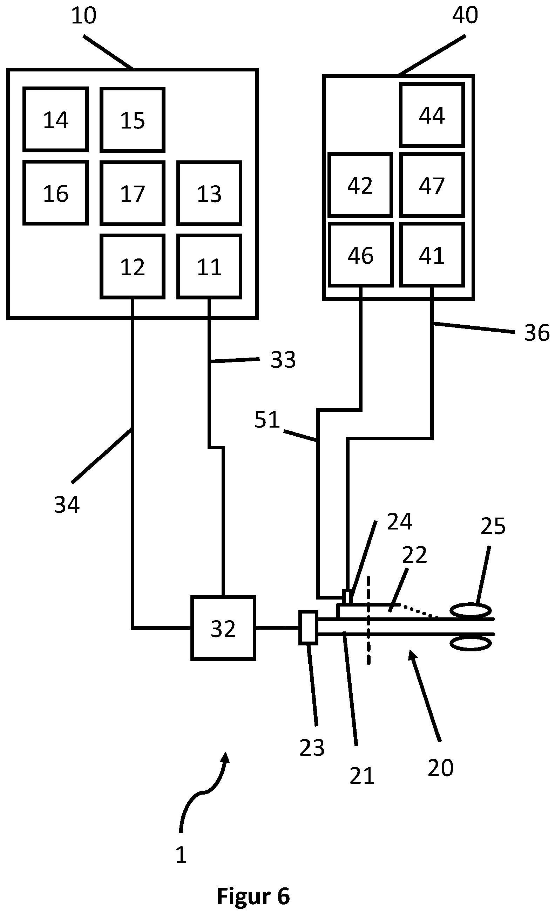

In Figur 6 ist eine beispielhafte Ausführungsform des Systems 1 dargestellt, bei welchem das Sprachgerät 40 unabhängig vom Beatmungsgerät 10 betrieben ist. In den wesentlichen Merkmalen entsprechen das Beatmungsgerät 10 und das Sprachgerät 40 den vorhergehend beschriebenen Ausführungsformen. In der gezeigten Ausführungsform wird steuert das Sprachgerät 40 insbesondere anhand der Sprachabsicht des Patienten ob bzw. in welchem Maße Sprachgas bereitgestellt wird. Eine Anpassung auf die Atemphasen des Patienten ist in dieser Ausführungsform nicht vorgesehen, kann aber auch zusätzlich realisiert werden, beispielsweise durch eine Übermittelung von Informationen über die Atemphasen des Patienten vom Beatmungsgerät 10 an das Sprachgerät 40.

Die Sprachabsicht des Patienten wird beispielsweise direkt über die Sprachröhre 22 erkannt. Dazu sind in der Sprachröhre 22 oder im Bereich der Sprachröhre 22 und/oder des Patienten und/oder des Sprachgerätes 40 und/oder des Beatmungsgerätes 10 entsprechende Sensoren angeordnet.

Beispielsweise ist eine Erkennung der Sprachabsicht über einen Beschleunigungssensor möglich. Dieser Beschleunigungssensor ist beispielhaft in der Sprachröhre 22 oder im Bereich der Sprachröhre 22 angeordnet und über die Sensorverbindung 51 mit dem Sprachgerät 40 verbunden. Eine Steuerung der Sprachgasförderungen bzw. der Zufuhr von Sprachgas zur Sprachröhre 22 wird dann entsprechend der Erkennung der Sprachabsicht geregelt. Bei Erkennen der Sprachabsicht stellt das Sprachgerät 40 automatisch einen ausreichenden Sprachgasstrom zur Verfügung um dem Patienten das Sprechen zu ermöglichen.

Alternativ oder ergänzend ist das Erkennen der Sprachabsicht auch über ein Mikrophon, welches im Bereich der Sprachröhre 22 und/oder des Patienten und/oder des Sprachgerätes 40 und/oder des Beatmungsgerätes 10 angeordnet ist, denkbar.

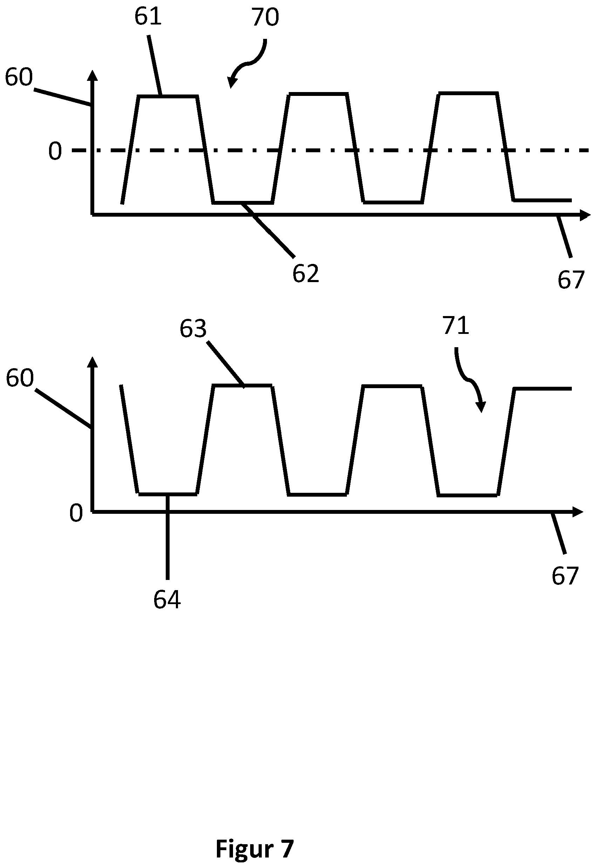

In Figur 7 ist beispielhaft und stark vereinfacht der zeitliche Verlauf des Flusses 60 vom Atemgasfluss 70, bereitgestellt durch das Beatmungsgerät 10, sowie vom Sprachgasfluss 71, bereitgestellt durch das Beatmungsgerät 10 (beispielhafte Ausführungsformen aus Figur 1 und/oder 2) oder das Sprachgerät 40 (beispielhafte Ausführungsformen aus Figuren 4 bis 6). Die X-Achse (Zeit 67) ist dabei für beide Verläufe gleich skaliert und umfasst den selben Zeitraum. Der Flussverlauf 70 des Atemgases folgt beispielhaft den Atemphasen des Patienten. Während der Inspiration 61 wird ein höherer Fluss registriert bzw. vorgegeben als während der Exspiration 62. Der Verlauf des Sprachgasflusses 71 verläuft dagegen umgekehrt, während der Exspiration 62 des Patienten findet also die Sprachunterstützung 63 (oder auch "Sprach-Modus") statt, ein erhöhter Fluss 60 an Sprachgas wird also bereitgestellt. Bei der in Figur 7 dargestellten Arbeitsweise wird der Sprachgasfluss 71 während der Inspiration 62 des Patienten nicht vollständig auf 0 (Null) abgesenkt, ein geringer Sprachgasfluss (und/oder Druck) wird auch während der Inspiration 62 des Patienten aufrechterhalten, beispielsweise als Standby-Modus 64. In der in Figur 7 gezeigten Arbeitsweise wechselt die Sprachunterstützung 63 periodisch mit dem Standby-Modus 64 im gleichen Maße wie der Atemgasfluss 70 zwischen Exspiration 61 und Inspiration 62 wechselt.

Der Sprachgasfluss 71 ist so dargestellt, dass ein positiver Wert einen Sprachgasfluss von der Gasquelle zum Patienten beschreibt. Je höher der Wert desto höher der Fluss, welcher beim Patienten bzw. in der Sprachröhre ankommt.

Das System 1 ist insbesondere dazu eingerichtet ein Rechteckprofil und/oder ein abnehmendes Flussprofil für den Sprachgasfluss 71 vorzugeben. Es kann zudem auch vorgesehen sein, dass das Flussprofil des Sprachgasflusses 71 zu Beginn und/oder zum Ende der Sprachgasförderung eine Rampe aufweist. In manchen Ausführungsformen ist das Flussprofil des Sprachgasflusses frei konfigurierbar. Es ist darauf hinzuweisen, dass das Flussprofil des Sprachgasflusses 71 bevorzugt nicht dem Flussprofil des Atemgasflusses des Patienten während der Exspiration 62 entspricht.

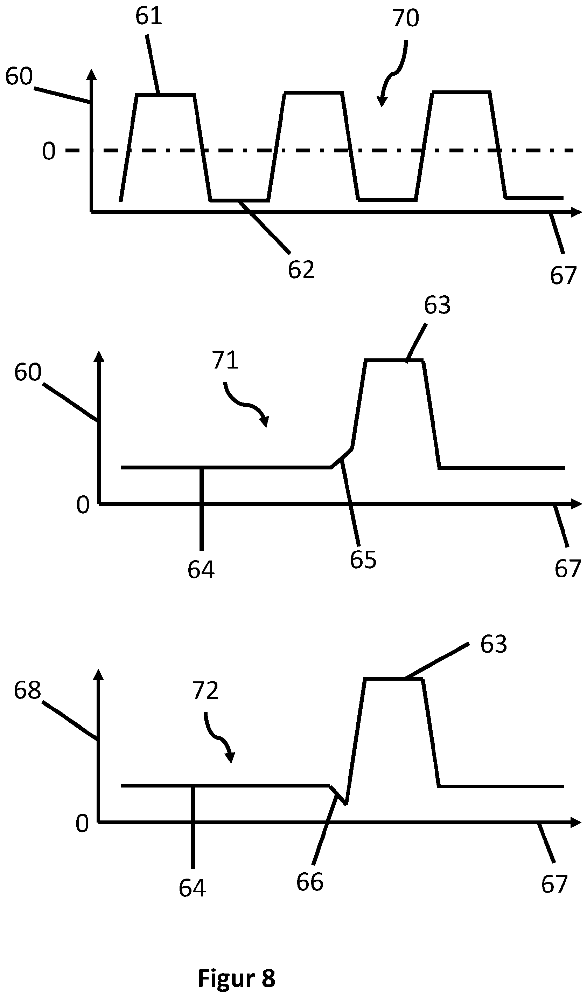

Figur 8 zeigt stark vereinfacht beispielhafte Fluss- bzw. Druckverläufe des Sprachgases bzw. des Atemgases. Der Atemgasfluss 70 folgt dabei den Atemphasen des Patienten, während der Inspiration 61 des Patienten wird ein positiver Fluss 60 durch das Beatmungsgerät 10 vorgegeben bzw. registriert, während der Exspiration 62 ein negativer Fluss, wobei ein positiver Fluss eine Atemgasströmung zum Patienten/in die Lunge darstellt und ein negativer Fluss eine Atemgasströmung vom Patienten/aus der Lunge darstellt. Beispielsweise wird die Sprachgasförderung so geregelt, dass während der Inspiration 61 des Patienten grundsätzlich keine Sprachunterstützung 63 erlaubt wird.

Beispielsweise wird ständig in einem Standby-Modus ein geringer Sprachgasfluss 71 bzw. Sprachgasdruck 72 vorgegeben, bis eine Sprachabsicht durch den Patienten festgestellt wird. Beispielsweise kann eine Sprachabsicht des Patienten dadurch festgestellt werden, dass ein Flussanstieg 65 des Sprachgasflusses 71 registriert wird. Wird eine Sprachabsicht festgestellt, so wird von dem Standby-Modus auf den Sprach-Modus bzw. die Sprachunterstützung 63 umgeschaltet, was beispielsweise eine Erhöhung des Sprachgasflusses 71 zur Folge hat und dem Patienten das Sprechen ermöglicht.

Alternativ oder ergänzend dazu ist auch eine Erkennung der Sprachabsicht durch einen Druckabfall 66 des Sprachgasdruckes 72 denkbar. Ebenso wie der Sprachgasfluss 71 wird der Sprachgasdruck 72 bei Erkennen einer Sprachabsicht des Patienten beim Umschalten vom Standby-Modus 64 auf die Sprachunterstützung 63 erhöht um dem Patienten das Sprechen zu ermöglichen. Das Umschalten von Sprachunterstützung 63 zurück in den Standby-Modus 64 kann beispielsweise anhand eines leichten Druckanstiegs (nicht dargestellt) in der Sprachröhre 22 erfolgen. Ein leichter Druckanstieg kann dabei durch die Bemühung des Patienten entstehen, die Glottis zu schließen. Alternativ oder ergänzend kann auch ein Abfall des Sprachgasflusses (nicht dargestellt) während der Sprachunterstützung 63 zu einem Umschalten in den Standby-Modus 64 führen.

Eine beispielhafte Ausführungsform des Patienteninterface 20 ist schematisch in Figur 9 dargestellt. Das Patienteninterface 20 umfasst eine Atemröhre 21 mit Atemanschluss 23 zum Anschluss an eine Atemgasleitung 33, beispielsweise über ein Y-Stück 32. Ferner umfasst das Patienteninterface 20 eine Sprachröhre 22 welche über den Sprachanschluss 24 mit einer Sprachgasleitung 36, beispielsweise von einem Beatmungsgerät 10 und/oder einem Sprachgerät 40 kommend, zumindest zeitweise mit Sprachgas versorgt wird. Die Sprachröhre 22 weist eine Sprachöffnung 26 auf durch welche das Sprachgas entweichen kann.

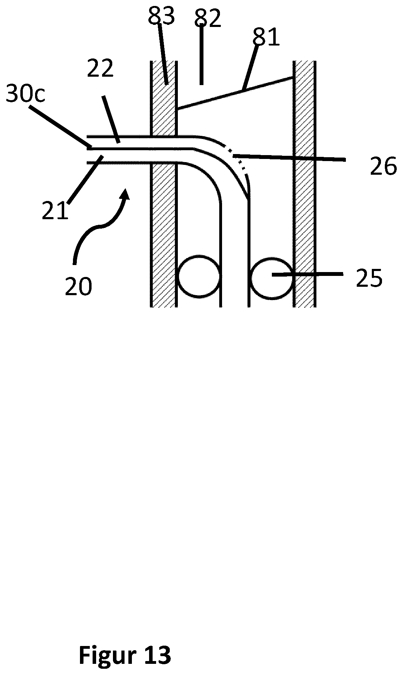

Das Patienteninterface 20 ist so eingerichtet und ausgebildet, dass die Atemröhre 21 und die Sprachröhre 22 zumindest teilweise in die Luftröhre 83 (siehe Figur 13) eines Patienten eingeführt werden können. Beispielhaft handelt es sich bei dem Patienteninterface 20 um eine Trachealkanüle, insbesondere als geblockte Ausführungen. Zumindest um die Atemröhre 21 ist abschnittsweise ein Dichtungselement 25, etwa ein Cuff bzw. ein Ballon, angeordnet, welcher die Luftröhre des Patienten um die Atemröhre 21 abdichtet. Durch die Atemröhre 21 ist dazu eingerichtet, Atemgas zu und/oder von der Lunge des Patienten zu leiten. Beispielsweise wird während der Inspiration des Patienten Atemgas durch die Atemröhre 21 zu der Lunge des Patienten geleitet und während der Exspiration Atemgas von der Lunge des Patienten weggeleitet. Das Dichtungselement 25 ist beispielsweise so angeordnet, dass Atemgas im Wesentlichen, also mit Ausnahme unvermeidbarer Leckagen, nur durch die Atemröhre 21 zu und von der Lunge des Patienten geleitet wird.

Die Sprachöffnung 26 der Sprachröhre 22 ist so angeordnet, dass das Sprachgas oberhalb des Dichtungselements 25 entweichen kann, also nicht in die Lunge des Patienten geleitet wird. Auch ist die Sprachöffnung 26 so angeordnet, dass sie zwischen der Glottis des Patienten und dem Dichtungselement 25 platziert wird. Bei geöffneter Glottis des Patienten kann also Sprachgas aus der Sprachröhre 22 durch die Glottis in den Mundraum des Patienten fließen.

In manchen Ausführungsformen des Patienteninterface 20 verlaufen Atemröhre 21 und Sprachröhre 22 zumindest abschnittsweise parallel.

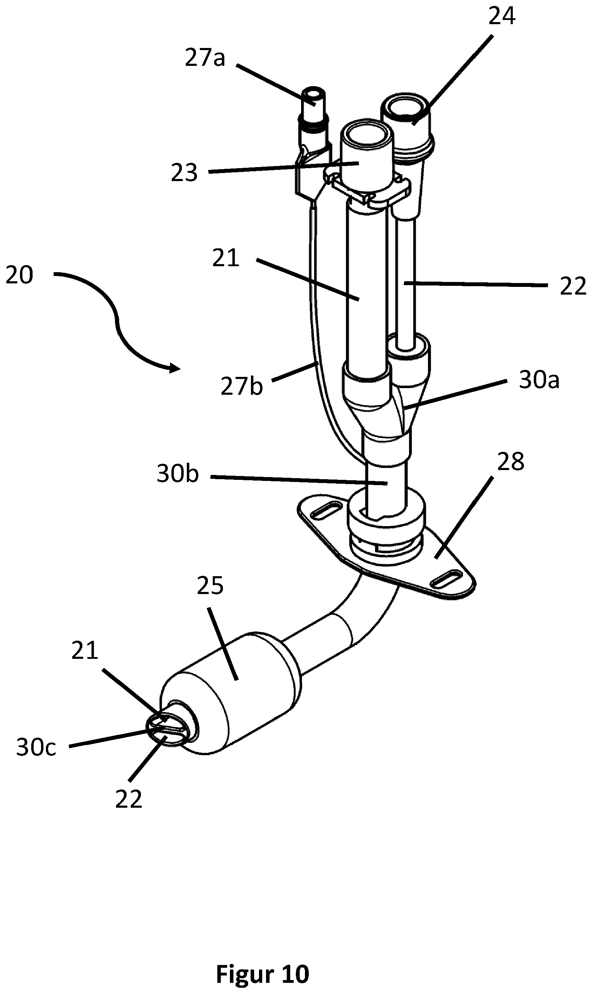

Eine beispielhafte Ausführungsform des Patienteninterface 20 als Trachealkanüle ist in den Figuren 10 bis 12 dargestellt. Die Trachealkanüle ist so eingerichtet, dass sie durch beispielsweise einen Luftröhrenschnitt unterhalb der Glottis in die Luftröhre des Patienten einführbar ist. Neben der Atemröhre 21 und der Sprachröhre 22 umfasst das beispielhafte Patienteninterface auch einen Ballonanschluss 27a mit einer Ballonleitung 27b um das als beispielhaft als Ballon ausgeführte Dichtungselement 25 zu expandieren und/oder den Druck in dem Dichtungselement 25 zu kontrollieren und gegebenenfalls zu regeln (erhöhen/erniedrigen).

Die Atemröhre 21 und die Sprachröhre 22 werden beginnend mit den Anschlüssen 23, 24 parallel geführt und in einem Y-Stück 30a in eine gemeinsame Röhre 30b geführt, wobei die Atemröhre 21 und die Sprachröhre 22 in der Röhre 30b durch eine Röhrenwand 30c voneinander getrennt werden, sodass kein Gas von der Atemröhre 21 in die Sprachröhre 22 oder umgekehrt übertreten kann. Die Röhre 30b ist beispielsweise ein Tubus mit zwei Lumen, wobei das eine Lumen für die Sprachröhre 22 dient und das andere Lumen für die Atemröhre 21 dient. Auch eine Anordnung der Anschlüsse 23, 24 direkt am Y-Stück 30a und/oder eine zumindest teilweise Integration der Anschlüsse 23, 24 in das Y-Stück 30a ist möglich. In manchen Ausführungsformen ist die Halterplatte 28 direkt am Y-Stück 30a angeordnet.

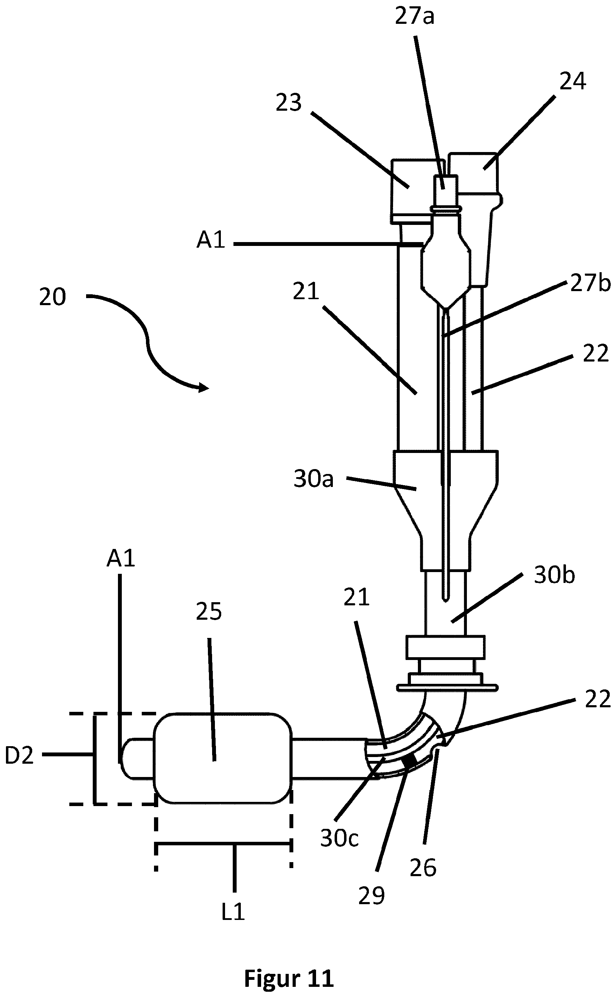

Wie in Figur 11 dargestellt ist die Sprachröhre 22 nach der Sprachöffnung 26 durch einen Röhrenverschluss 29 blockiert, sodass keine Sprachgas hinter das Dichtungselement 25 und in die Lunge des Patienten gelangt. Die Röhre 30b weist beispielsweise einen Knick/Biegung, zum Beispiel in einem 90° Winkel, auf. Die Sprachöffnung 26 ist beispielsweise in dem Knick/Biegung ausgebildet. Durch die Sprachöffnung 26 kann Sprachgas aus der Sprachröhre 22 in die Luftröhre des Patienten geleitet werden, sodass diese durch eine geöffnete Glottis in den Mundraum des Patienten entweichen kann. Das Dichtungselement 25 ist beispielsweise so angeordnet, dass es um die Röhre 30b verläuft.

Das in Figuren 10 bis 12 beispielhafte dargestellte Patienteninterface 20 umfasst weiter eine Halterplatte 28 um das Patienteninterface 20 am Hals des Patienten zu fixieren. Dazu kann die Halterplatte 28 beispielsweise mit einer Bänderung verbunden werden, welche um den Hals des Pateinten geführt werden kann und das Patienteninterface 20 in Position halten kann.

Beim Verlauf des Patienteninterfaces (20) kann beispielsweise der Anfang in den Anschlüssen für Sprachgas (Sprachanschluss 24) und Atemgas (Atemanschluss 23) gesehen werden. In der beispielhaften Ausführungsform ist nachfolgend das Y-Stück 30a angeordnet, welches die Atemröhre 21 und die Sprachröhre 22 in eine Röhre 30b zusammenführt, wobei die Sprachröhre 22 von der Atemröhre 21 durch eine Röhrenwand 30c getrennt werden Die Röhre 30b kann also als Röhre mit zwei Lumen verstanden werden, wobei ein Lumen das Sprachgas leitet und ein Lumen das Atemgas. Ist das Patienteninterface 20, wie dargestellt, eine Trachealkanüle, knickt die Röhre 30b nach der Halterplatte 28 in einem Winkel zwischen 45° und 135° ab. In diesem Knick/Biegung ist die Sprachöffnung 26 angeordnet, über welche das Sprachgas aus der Sprachröhre 22 entweichen kann. Im weiteren Verlauf ist die Sprachröhre 22, nach der Sprachöffnung 26, durch einen Röhrenverschluss 29 verschlossen, wie in Figur 11 dargestellt. Vor dem Ende der Röhre 30b befindet sich an der Außenseite das Dichtelement 25. Die Atemröhre 21 ist zum Ende offen, sodass Atemgas durch die Atemröhre 21 zu/von der Lunge des Patienten geleitet werden kann.

In manchen Ausführungsformen weist die Röhre 30b eine 180° Wendung auf, wobei die Halterplatte 28 mittig in der Wendung angeordnet ist. Alternativ weist die Röhre vor der Halterplatte 28 einen Knick mit einem Winkel von 45° bis 90° auf und hinter der Halterplatte 28 ebenfalls einen Knick mit einem Winkel von 45° bis 90° auf. So verlaufen der Abschnitt der Röhre 30b, welcher außerhalb des Halses des Patienten geführt wird und der Abschnitt, welcher innerhalb des Halses des Patienten geführt wird, zumindest abschnittsweise parallel.

Das Patienteninterface 20 besteht beispielhaft zumindest teilweise aus PVC (Polyvinylchlorid), PUR (Polyurethan), PE (Polyethylen), EVA (Ethylen-Vinylacetat-Copolymer) und/oder ABS (Acrylnitril-Butadien-Styrol-Copolymer). Beispielhaft wird die Röhre 30c aus PVC gefertigt.

Der Durchmesser der Röhre 30b beträgt zwischen 10 mm und 20 mm, beispielhaft zwischen 11 mm und 15 mm. Der Durchmesser D2 des Dichtungselements 25, beispielhaft als annähernd zylindrischer Cuff ausgeführt, beträgt zwischen 20 mm und 40 mm, beispielhaft zwischen 24 mm und 30 mm und/oder zwischen 26 mm und 28 mm. Die Länge L1 des Dichtungselements beträgt zwischen 30 mm und 50 mm, beispielhaft zwischen 35 mm und 45 mm. Die Gesamtlänge in einem gestreckten Zustand von A1, beispielsweise durch den Übergang vom Atemanschluss 23 zu der Atemröhre 21 gekennzeichnet, bis zu A2, welches das Ende der Röhre 30b darstellt, beträgt zwischen 100 mm und 250 mm, beispielsweise zwischen 150 mm und 200 mm. Die Länge des Abschnittes der Röhre hinter der Halterplatte 28 beträgt, zwischen A2 und A3, beispielsweise im gebogenen Zustand, beträgt zwischen 50 mm und 110 mm, in manchen Ausführungsformen zwischen 70 mm und 100 mm. Das Ende A1 der Röhre 30b ist beispielsweise abgeschrägt.

Das Patienteninterface 20 in Figur 13 ist beispielhaft eine Trachealkanüle, welche unterhalb der Glottis 81 des Patienten beispielsweise durch einen Luftröhrenschnitt in die Luftröhre 82 des Patienten eingeführt ist. Das Dichtungselement 25 ist so angeordnet, dass die Luftröhre 82 zumindest um die Atemröhre 21 unterhalb der Glottis 81 des Patienten dichtet. Die Sprachöffnung 26 der Sprachröhre 22 ist so angeordnet, dass Sprachgas aus der Sprachröhre 22 unterhalb der Glottis 81 aber oberhalb des Dichtungselement 25 in die Luftröhre des Patienten geleitet wird. Öffnet der Patient die Glottis 81, so kann das Sprachgas in den Mundraum des Patienten strömen und dem Patient wird das Sprechen ermöglicht.

### Bezugszeichenliste

- 1: System
- 10: Beatmungsgerät
- 11: Atemgasquelle
- 12: Atemabluft
- 13: Sprachsteuerung
- 14: Steuereinheit
- 15: Auswerteeinheit
- 16: Sensoreinheit
- 17: Benutzerschnittstelle
- 18: Schnittstelle
- 20: Interface
- 21: Atemröhre
- 22: Sprachröhre
- 23: Atemanschluss
- 24: Sprachanschluss
- 25: Dichtungselement
- 26: Sprachöffnung
- 27a: Ballonanschluss
- 27b: Ballonleitung
- 28: Halterplatte
- 29: Röhrensverschluss
- 30a: Y-Stück
- 30b: Röhre
- 30c: Röhrenwand
- 31: Ventil
- 32: Y-Stück
- 33: Atemgasleitung
- 34: Atemgasleitung
- 35: Steuerleitung
- 36: Sprachgasleitung
- 37: (3/2 Wege) Ventil
- 38: Steuerleitung
- 39: Abluft
- 40: Sprachgerät
- 41: (Sprach-)Gasquelle
- 42: Steuereinheit
- 43: Schnittstelle
- 44: Benutzerschnittstelle
- 45: Ventilsteuerung
- 46: Sensoreinheit
- 47: Auswerteeinheit
- 51: Sensorverbindung
- 60: Fluss
- 61: Inspiration
- 62: Exspiration
- 63: Sprachunterstützung
- 64: Standby
- 65: Flussanstieg
- 66: Druckabfall
- 67: Zeit
- 68: Druck
- 70: Atemgasfluss
- 71: Sprachgasfluss
- 72: Sprachgasdruck
- 81: Glottis
- 82: Luftröhre
- 83: Hals

## Patentansprüche

1. System (1) zur Sprachunterstützung eines Patienten, umfassend zumindest ein Beatmungsgerät (10) und ein Patienteninterface (20), wobei das Beatmungsgerät (10) zumindest eine steuerbare Atemgasquelle (11) umfasst und dazu ausgebildet ist die Atemphasen, zumindest aber Inspiration und Exspiration, des Patienten zu erkennen und wobei das Patienteninterface (20) zumindest eine Sprachröhre (22) und eine Atemröhre (21) aufweist und dazu eingerichtet ist über die Sprachröhre (22) Sprachgas zum Patienten zu leiten und über die Atemröhre (21) Atemgas zum und/oder vom Patienten zu leiten, wobei das System (1) dazu eingerichtet ist, dem Patienten zumindest zeitweise in einem Sprach-Modus (63) Sprachgas zur Verfügung zu stellen, um das Sprechen zu ermöglichen, **dadurch gekennzeichnet, dass** das System (1) dazu eingerichtet und ausgebildet ist, in einen Standby-Modus (64) zu schalten, während der Sprach-Modus (63) nicht aktiv ist, wobei im Standby-Modus (64) ein Druck und/oder Fluss an Sprachgas in der Sprachröhre (22) anliegt, welcher geringer ist als der Druck und/oder Fluss im Sprach-Modus (63), wobei das System (1) dazu eingerichtet und ausgebildet ist eine Sprachabsicht des Patienten zu erkennen und nur dann in den Sprach-Modus (63) schaltet, wenn eine Sprachabsicht des Patienten erkannt wird.

2. System (1) nach Anspruch 1, **dadurch gekennzeichnet, dass** das Patienteninterface (20) eine Trachealkanüle ist.

3. System (1) nach zumindest einem der vorhergehenden Ansprüche, **dadurch gekennzeichnet, dass** das System (1) dazu eingerichtet ist nur während der Exspiration des Patienten in den Sprach-Modus (63) zu schalten.

4. System (1) nach zumindest einem der vorhergehenden Ansprüche, **dadurch gekennzeichnet, dass** das System (1) dazu eingerichtet und ausgebildet ist, während des Standby-Modus (64) einen Druck und/oder Fluss an Sprachgas vorzugeben, bei welchem der Patient die Glottis schließen kann bzw. geschlossen halten kann.

5. System (1) nach zumindest einem der vorhergehenden Ansprüche, **dadurch gekennzeichnet, dass** das System (1) dazu eingerichtet ist, dass eine Sprachfunktion aktiviert und deaktiviert werden kann, wobei eine Schaltung in den Sprach-Modus (63) nur bei aktivierter Sprachfunktion ermöglicht wird.

6. System (1) nach Anspruch 5, **dadurch gekennzeichnet, dass** das System (1) dazu eingerichtet ist, dass bei deaktivierter Sprachfunktion kein Sprachgas zur Sprachröhre (22) gefördert wird.

7. System (1) nach zumindest einem der vorhergehenden Ansprüche, **dadurch gekennzeichnet, dass** das System (1) dazu eingerichtet ist, periodisch zwischen dem Standby-Modus (64) und dem Sprach-Modus (63) umzuschalten, wobei das System (1) während der Inspiration des Patienten in den Standby-Modus (64) schaltet und während der Exspiration des Patienten in den Sprach-Modus (63) schaltet.

8. System (1) nach Anspruch 7, **dadurch gekennzeichnet, dass** das System (1) dazu eingerichtet und ausgebildet ist, die Sprachabsicht des Patienten anhand eines Druckabfalls und/oder Flussanstiegs des Sprachgases in der Sprachröhre (22) zu erkennen.

9. System (1) nach einem der Ansprüche 7 oder 8, **dadurch gekennzeichnet, dass** das System (1) dazu eingerichtet und ausgebildet ist, die Sprachabsicht des Patienten über zumindest einen Beschleunigungssensor und/oder zumindest ein Mikrophon zu erkennen.

10. System (1) nach zumindest einem der vorhergehenden Ansprüche, **dadurch gekennzeichnet, dass** die Atemgasquelle (11) dazu eingerichtet und ausgebildet ist, Sprachgas und Atemgas zu fördern.

11. System (1) nach zumindest einem der vorhergehenden Ansprüche, **dadurch gekennzeichnet, dass** das System (1) eine Sprachsteuerung (13) und mindestens ein Steuerventil (31, 37) umfasst, wobei die Sprachsteuerung (13) dazu eingerichtet ist das Steuerventil (31, 37) zu steuern, wobei das Steuerventil (31. 37) zumindest mit der Sprachröhre (22) des Patienteninterface (20) gasleitend verbunden ist, wobei das Steuerventil (31) mit einem Y-Stück (32) verbunden ist, wobei das Y-Stück (32) zumindest mit der Atemröhre (21) des Patienteninterface (20) verbunden ist und durch das Steuerventil (31) sowohl Atemgas als auch Sprachgas geleitet wird.

12. System (1) nach Anspruch 11, **dadurch gekennzeichnet, dass** das System (1) eingerichtet ist, das Ventil (31, 37) so zu schalten, dass zumindest im Sprach-Modus (63) Sprechgas zur Sprachröhre (22) gefördert wird.

13. System (1) nach einem der Ansprüche 11 oder 12, **dadurch gekennzeichnet, dass** das Ventil (31, 37) so schaltbar ist, dass während des Standby-Modus (64) ein Druck und/oder Fluss an Sprachgas in der Sprachröhre (22) aufrechterhalten wird, wobei der Druck und/oder Fluss geringer ist als der Druck und/oder Fluss des Sprachgases im Sprach-Modus (63).

14. System (1) nach zumindest einem der vorhergehenden Ansprüche, **dadurch gekennzeichnet, dass** das System (1) neben dem Beatmungsgerät (10) zumindest ein Sprachgerät (40) umfasst, wobei das Sprachgerät (40) zumindest eine Gasquelle (41) umfasst, welche dazu eingerichtet ist zumindest zeitweise Sprachgas zu fördern.

15. System (1) nach Anspruch 14, **dadurch gekennzeichnet, dass** das Sprachgerät (40) eingerichtet zwischen dem Sprach-Modus (63) und dem Standby-Modus (64) umzuschalten.

16. System (1) nach einem der Ansprüche 14 oder 15, **dadurch gekennzeichnet, dass** das Sprachgerät (40) mit dem Beatmungsgerät (10) verbunden ist, wobei das Sprachgerät (40) an das Beatmungsgerät (10) ankoppelbar ist.

17. System (1) nach zumindest einem der Ansprüche 14 bis 16, **dadurch gekennzeichnet, dass** das Sprachgerät (40) dazu eingerichtet ist Informationen zu den Atemphasen des Patienten vom Beatmungsgerät (10) zu empfangen und anhand dieser Informationen den Sprachgasstrom zu steuern.

18. System (1) nach zumindest einem der Ansprüche 14 bis 17, **dadurch gekennzeichnet, dass** das Sprachgerät (40) dazu eingerichtet ist die Atemphasen des Patienten, zumindest die Inspiration und Exspiration, zu erkennen und anhand der Atemphasen in den Sprach-Modus (63) oder den Standby-Modus (64) zu schalten.

19. System (1) nach zumindest einem der Ansprüche 14 bis 18, **dadurch gekennzeichnet, dass** das Sprachgerät (40) dazu eingerichtet und ausgebildet ist eine Sprachabsicht des Patienten zu erkennen und bei einer erkannten Sprachabsicht in den Sprach-Modus (63) zu schalten.

20. Verfahren zur Steuerung einer Sprachgasförderung, **dadurch gekennzeichnet, dass** ein Sprachgasfluss (71) und/oder Sprachgasdruck (72) in einem Sprach-Modus (63) auf einen ersten Wert vorgegeben wird und in einem Standby-Modus (64) auf einen zweiten Wert eingestellt wird, wobei der erste Wert höher ist als der zweite Wert und der erste Wert so eingestellt wird, dass einem Anwender das Sprechen ermöglicht wird und der Sprachgasfluss (71) und/oder Sprachgasdruck (72) in dem Standby-Modus (64) nicht vollständig auf Null abgesenkt wird, wobei die Umschaltung von Standby-Modus in den Sprach-Modus die Erkennung einer Sprachabsicht des Anwenders erfordert

## Claims

1. A system (1) for assisting with a patient's speech, comprising at least a ventilator (10) and a patient interface (20), wherein the ventilator (10) comprises at least a controllable breathing gas source (11) and is designed to recognize the patient's breathing phases, but at least inhalation and exhalation, and wherein the patient interface (20) has at least a speaking tube (22) and a breathing tube (21) and is configured to convey speech gas to the patient via the speaking tube (22) and to convey breathing gas to and/or from the patient via the breathing tube (21), wherein the system (1) is configured to make speech gas available to the patient at least intermittently in a speaking mode (63) in order to make speaking possible, **characterized in that** the system (1) is configured and designed to switch into a standby mode (64) while the speaking mode (63) is not active, wherein there is a pressure and/or flow of speech gas in the speaking tube (22) in the standby mode (64) that is lower than the pressure and/or flow in the speaking mode (63), wherein the system (1) is configured and designed to recognize the patient's intention to speak and only then switches into the speaking mode (63) when the patient's intention to speak is recognized.

2. The system (1) according to claim 1, **characterized in that** the patient interface (20) is a tracheal cannula.

3. The system (1) according to at least one of the preceding claims, **characterized in that** the system (1) is configured to switch into the speaking mode (63) only when the patient exhales.

4. The system (1) according to at least one of the preceding claims, **characterized in that** the system (1) is configured and designed to specify a pressure and/or flow of speech gas during the standby mode (64) at which the patient can close or else keep the glottis closed.

5. The system (1) according to at least one of the preceding claims, **characterized in that** the system (1) is configured such that a speaking function can be activated and deactivated, wherein switching into the speaking mode (63) is only made possible when the speaking function is activated.

6. The system (1) according to claim 5, **characterized in that** the system (1) is configured such that no speech gas is conveyed to the speaking tube (22) when the speaking function is deactivated.

7. The system (1) according to at least one of the preceding claims, **characterized in that** the system (1) is configured to periodically switch between the standby mode (64) and the speaking mode (63), wherein the system (1) switches into the standby mode (64) when the patient inhales and switches into the speaking mode (63) when the patient exhales.

8. The system (1) according to claim 7, **characterized in that** the system (1) is configured and designed to recognize the patient's intention to speak based on a pressure drop and/or flow increase of the speech gas in the speaking tube (22).

9. The system (1) according to one of claims 7 or 8, **characterized in that** the system (1) is configured and designed to recognize the patient's intention to speak using at least one acceleration sensor and/or at least one microphone.

10. The system (1) according to at least one of the preceding claims, **characterized in that** the breathing gas source (11) is configured and designed to deliver speech gas and breathing gas.

11. The system (1) according to at least one of the preceding claims, **characterized in that** the system (1) comprises a speech controller (13) and at least one control valve (31, 37), wherein the speech controller (13) is configured to control the control valve (31, 37), wherein the control valve (31, 37) is connected in a gas-transporting manner at least to the speaking tube (22) of the patient interface (20), wherein the control valve (31) is connected to a Y-piece (32), wherein the Y-piece (32) is connected at least to the breathing tube (21) of the patient interface (20) and both breathing gas and speech gas are transported through the control valve (31).

12. The system (1) according to claim 11, **characterized in that** the system (1) is configured to switch the valve (31, 37) in such a way that speech gas is delivered to the speaking tube (22) at least in the speaking mode (63).

13. The system (1) according to one of claims 11 or 12, **characterized in that** the valve (31, 37) is switchable in such a way that, during the standby mode (64), a pressure and/or flow of speech gas in the speaking tube (22) is maintained, wherein the pressure and/or flow is lower than the pressure and/or flow of the speech gas in the speaking mode (63).

14. The system (1) according to at least one of the preceding claims, **characterized in that** the system (1) comprises at least a speaking device (40) in addition to the ventilator (10), wherein the speaking device (40) comprises at least a gas source (41) which is configured to at least intermittently deliver speech gas.

15. The system (1) according to claim 14, **characterized in that** the speaking device (40) is configured to switch between the speaking mode (63) and the standby mode (64).

16. The system (1) according to one of claims 14 or 15, **characterized in that** the speaking device (40) is connected to the ventilator (10), wherein the speaking device (40) is couplable to the ventilator (10).

17. The system (1) according to at least one of claims 14 to 16, **characterized in that** the speaking device (40) is configured to receive information relating to the patient's breathing phases from the ventilator (10) and to control the speech gas flow based on this information.

18. The system (1) according to at least one of claims 14 to 17, **characterized in that** the speaking device (40) is configured to recognize the patient's breathing phases, at least the inhalation and exhalation, and to switch into the speaking mode (63) or standby mode (64) based on the breathing phases.

19. The system (1) according to at least one of claims 14 to 18, **characterized in that** the speaking device (40) is configured and designed to recognize the patient's intention to speak and to switch into the speaking mode (63) when an intention to speak is recognized.

20. A method for controlling speech gas delivery, **characterized in that** a speech gas flow (71) and/or speech gas pressure (72) is specified at a first value in a speaking mode (63) and is set to a second value in a standby mode (64), wherein the first value is higher than the second value and the first value is set such that it is made possible for a user to speak and the speech gas flow (71) and/or speech gas pressure (72) in the standby mode (64) is not reduced entirely to zero, wherein the switching from standby mode into the speaking mode requires recognition of the user's intention to speak.

## Revendications

1. Système (1) d'assistance vocale pour un patient, comprenant au moins un ventilateur (10) et une interface patient (20), dans lequel le ventilateur (10) comprend au moins une source de gaz respiratoire manœuvrable (11) et est conçu pour détecter les phases respiratoires du patient, au moins l'inspiration et l'expiration, et dans lequel l'interface patient (20) comporte au moins un tube phonatoire (22) et un tube respiratoire (21) et est agencée pour acheminer, via le tube phonatoire (22), du gaz de phonation vers le patient et, via le tube respiratoire (21), du gaz respiratoire vers et/ou depuis le patient, dans lequel le système (1) est agencé pour fournir au patient, au moins temporairement dans un mode phonatoire (63), du gaz de phonation afin de permettre la parole, **caractérisé en ce que** le système (1) est agencé et conçu pour commuter dans un mode veille (64), pendant que le mode phonatoire (63) n'est pas actif, dans lequel en mode veille (64) une pression et/ou un flux de gaz de phonation est présent(e) dans le tube phonatoire (22), laquelle/lequel est plus faible que la pression et/ou le flux en mode phonatoire (63), dans lequel le système (1) est agencé et configuré pour détecter une intention de parole du patient et ne commute en mode phonatoire (63) que si une intention de parole du patient est détectée.

2. Système (1) selon la revendication 1, **caractérisé en ce que** l'interface patient (20) est une canule trachéale.

3. Système (1) selon au moins l'une des revendications précédentes, **caractérisé en ce que** le système (1) est agencé pour commuter en mode phonatoire (63) uniquement pendant l'expiration du patient.

4. Système (1) selon au moins l'une des revendications précédentes, **caractérisé en ce que** le système (1) est agencé et conçu pour appliquer, pendant le mode veille (64), une pression et/ou un flux de gaz de phonation permettant au patient de fermer la glotte ou respectivement de la maintenir fermée.

5. Système (1) selon au moins l'une des revendications précédentes, **caractérisé en ce que** le système (1) est agencé de telle sorte qu'une fonction de parole peut être activée et désactivée, la commutation en mode phonatoire (63) n'étant possible que lorsque la fonction de parole est activée.

6. Système (1) selon la revendication 5, **caractérisé en ce que** le système (1) est agencé de sorte qu'aucun gaz de phonation n'est distribué vers le tube phonatoire (22) lorsque la fonction de parole est désactivée.

7. Système (1) selon au moins l'une des revendications précédentes, **caractérisé en ce que** le système (1) est agencé pour commuter périodiquement entre le mode veille (64) et le mode phonatoire (63), le système (1) commutant en mode veille (64) pendant l'inspiration du patient et en mode phonatoire (63) pendant l'expiration du patient.

8. Système (1) selon la revendication 7, **caractérisé en ce que** le système (1) est agencé et conçu pour détecter l'intention de parole du patient au moyen d'une baisse de pression et/ou d'une augmentation du flux de gaz de phonation dans le tube phonatoire (22).

9. Système (1) selon l'une des revendications 7 ou 8, **caractérisé en ce que** le système (1) est agencé et conçu pour détecter l'intention de parole du patient via au moins un accéléromètre et/ou au moins un microphone.

10. Système (1) selon au moins l'une des revendications précédentes, **caractérisé en ce que** la source de gaz respiratoire (11) est agencée et conçue pour distribuer du gaz de phonation et du gaz respiratoire.

11. Système (1) selon au moins l'une des revendications précédentes, **caractérisé en ce que** le système (1) comprend une commande phonatoire (13) et au moins une valve de commande (31, 37), la commande phonatoire (13) étant agencée pour commander la valve de commande (31, 37), la valve de commande (31, 37) étant reliée par liaison gazeuse au moins au tube phonatoire (22) de l'interface patient (20), la valve de commande (31) étant reliée à une pièce en Y (32), la pièce en Y (32) étant reliée au moins au tube respiratoire (21) de l'interface patient (20), et autant le gaz respiratoire que le gaz de phonation étant acheminés à travers la valve de commande (31).

12. Système (1) selon la revendication 11, **caractérisé en ce que** le système (1) est agencé pour commuter la valve (31, 37) de sorte qu'au moins en mode phonatoire (63), du gaz de phonation est distribué vers le tube phonatoire (22).

13. Système (1) selon l'une des revendications 11 ou 12, **caractérisé en ce que** la valve (31, 37) peut être commutée de telle sorte que, pendant le mode veille (64), une pression et/ou un flux de gaz de phonation sont maintenus dans le tube phonatoire (22), la pression et/ou le flux étant inférieurs à la pression et/ou au flux du gaz de phonation en mode phonatoire (63).

14. Système (1) selon au moins l'une des revendications précédentes, **caractérisé en ce que** le système (1) comprend, outre le ventilateur (10), au moins un appareil phonatoire (40), lequel appareil phonatoire (40) comprend au moins une source de gaz (41) qui est agencée pour distribuer du gaz de phonation au moins temporairement.

15. Système (1) selon la revendication 14, **caractérisé en ce que** l'appareil phonatoire (40) est agencé pour commuter entre le mode phonatoire (63) et le mode veille (64).

16. Système (1) selon l'une des revendications 14 ou 15, **caractérisé en ce que** l'appareil phonatoire (40) est relié au ventilateur (10), l'appareil phonatoire (40) pouvant être couplé au ventilateur (10).

17. Système (1) selon au moins l'une des revendications 14 à 16, **caractérisé en ce que** l'appareil phonatoire (40) est agencé pour recevoir du ventilateur (10) des informations sur les phases respiratoires du patient et pour contrôler le débit de gaz de phonation au moyen de ces informations.

18. Système (1) selon au moins l'une des revendications 14 à 17, **caractérisé en ce que** l'appareil phonatoire (40) est agencé pour détecter les phases respiratoires du patient, au moins l'inspiration et l'expiration, et pour commuter en mode phonatoire (63) ou en mode veille (64) au moyen des phases respiratoires.

19. Système (1) selon au moins l'une des revendications 14 à 18, **caractérisé en ce que** l'appareil phonatoire (40) est agencé et conçu pour détecter une intention de parole du patient et pour commuter en mode phonatoire (63) lorsqu'une intention de parole est détectée.

20. Procédé de commande d'une distribution de gaz de phonation **caractérisé en ce qu'**un flux de gaz de phonation (71) et/ou une pression de gaz de phonation (72) sont réglés sur une première valeur en mode phonatoire (63) et sur une seconde valeur en mode veille (64), la première valeur étant supérieure à la seconde et la première valeur est réglée de manière à permettre la parole à un utilisateur, et à ce que le flux de gaz de phonation (71) et/ou la pression de gaz de phonation (72) ne soient pas complètement réduit(e)s à zéro en mode veille (64), la commutation du mode veille au mode phonatoire nécessitant la détection d'une intention de parole de l'utilisateur.
